# EUROPÄISCHE PATENTSCHRIFT

(11) **EP 1 630 175 B1**
(45) Veröffentlichungstag und Bekanntmachung des Hinweises auf die Patenterteilung: **11.06.2008**
(21) Anmeldenummer: 05018249.2
(22) Anmeldetag: 23.08.2005
(51) Int. Cl.: C07K 16/36

(54) **Gegen den Marburg I Polymorphismus der Faktor VII aktivierenden Protease (FSAP) gerichtete Antikörper, ihre Herstellung und Verwendung**
Antibodies directed against Marburg I polymorphism of factor VII activating protease (FSAP), production and use thereof
Anticorps dirigés contre le polymorphisme Marburg I de la protéase activatrice du facteur VII (FSAP), leur production et utilisation

(30) Priorität: 24.08.2004 DE 102004041104; 06.06.2005 DE 102005026163
(43) Veröffentlichungstag der Anmeldung: 01.03.2006
(73) Patentinhaber: Dade Behring Marburg GmbH, 35001 Marburg (DE)
(72) Erfinder: Althaus, Harald, 35083 Wetter (DE); Schwarz, Herbert, 35102 Lohra (DE); Fischer, Bodo, 35039 Marburg (DE); Wissel, Thomas, Dr., 35094 Lahntal-Sarnau (DE)

(56) Entgegenhaltungen:
- US-A1- 2002 142 316
- SHANGARY SANJEEV ET AL: "Peptides derived from BH3 domains of Bcl-2 family members: A comparative analysis of inhibition of Bcl-2, Bcl-xL and Bax oligomerization, induction of cytochrome c release, and activation of cell death" BIOCHEMISTRY, Bd. 41, Nr. 30, 30. Juli 2002 (2002-07-30), Seiten 9485-9495, XP002359834 ISSN: 0006-2960
- BURSTEIN ETHAN S ET AL: "Amino acid side chains that define muscarinic receptor/G-protein coupling: Studies of the third intracellular loop" JOURNAL OF BIOLOGICAL CHEMISTRY, Bd. 271, Nr. 6, 1996, Seiten 2882-2885, XP002359835 ISSN: 0021-9258
- ROEMISCH J ET AL: "QUANTIATION OF THE FACTOR VII- AND SINGLE-CHAIN PLASMINOGEN ACTIVATOR-ACTIVATING PROTEASE IN PLASMAS OF HEALTHY SUBJECTS" BLOOD COAGULATION & FIBRINOLYSIS, RAPID COMMUNICATIONS, OXFORD,OXFORD, GB, Bd. 12, Nr. 5, Juli 2001 (2001-07), Seiten 375-383, XP009013812 ISSN: 0957-5235
- WILLEIT J ET AL: "MARBURG I POLYMORPHISM OF FACTOR VII-ACTIVATING PROTEASE A PROMINENT RISK PREDICTOR OF CAROTID STENOSIS" CIRCULATION, AMERICAN HEART ASSOCIATION, DALLAS, TX, US, Bd. 107, Nr. 5, 11. Februar 2003 (2003-02-11), Seiten 667-670, XP009013816 ISSN: 0009-7322

### SEQUENCE LISTING

<110> Dade Behring Marburg GmbH
<120> Gegen den Marburg I Polymorphismus der Faktor VII aktivierenden Protease (FSAP) gerichtete Antikörper, ihre Herstellung und Verwendung
<130> 2004/B002J-Ma1261
<150> DE 10 2004 041 104.2
   <151> 2004-08-24
<150> DE 10 2005 026 163.9
   <151> 2005-06-06
<160> 6
<170> PatentIn version 3.1
<210> 1
   <211> 5
   <212> PRT
   <213> Artificial Sequence
<220>
   <223> Oligopeptide
<400> 1
   Glu Cys Glu Lys Arg
   1               5
<210> 2
   <211> 25
   <212> PRT
   <213> Artificial Sequence
<220>
   <223> Oligopeptide
<400> 2
   Tyr Val Tyr Gly Ile Val Ser Trp Gly Leu Glu Cys Glu Lys Arg Pro
   1               5                   10                  15
   Gly Val Tyr Thr Gln Val Tyr Lys Phe
               20                  25
<210> 3
   <211> 13
   <212> PRT
   <213> Artificial Sequence
<220>
   <223> Oligopeptide
<400> 3
   Ser Trp Gly Leu Glu Cys Glu Lys Arg Pro Gly Val Tyr
   1               5                   10
<210> 4
   <211> 5
   <212> PRT
   <213> Artificial Sequence
<220>
   <223> Oligopeptide
<400> 4
   Glu Glu Phe His Glu
   1 5
<210> 5
   <211> 15
   <212> PRT
   <213> Artificial Sequence
<220>
   <223> Oligopeptide
<400> 5
   Gln Asp Leu Lys Lys Glu Glu Phe His Glu Gln Ser Phe Arg Val
   1 5 10 15
<210> 6
   <211> 13
   <212> PRT
   <213> Artificial Sequence
<220>
   <223> Oligopeptide
<400> 6
   Ser Trp Gly Leu Glu Cys Gly Lys Arg Pro Gly Val Tyr
   1 5 10

## Beschreibung

Die Erfindung betrifft gegen den Marburg I Polymorphismus der Blutgerinnungsfaktor VII aktivierenden Protease (FSAP) gerichtete Antikörper, ihre Herstellung und Verwendung.

Die Faktor VII aktivierende Protease (FSAP) ist eine plasmatische Serinprotease, die neben ihrer Eigenschaft den Blutgerinnungsfaktor VII zu aktivieren, außerdem über Plasminogenaktivator-, also Prourokinase-aktivierende Eigenschaften verfügt [Roemisch et al. (1999) Haemostasis 76: 292-299; EP 952 215 A2]. Dies deutet darauf hin, dass FSAP sowohl eine Rolle in der Blutgerinnungskaskade als auch im Fibrinolysesystem spielt.

FSAP liegt im Humanplasma in einer Konzentration von ca. 12 µg/ml vor und kann über Autokatalyse vom einkettigen Proenzym in die aktive zweikettige Protease überführt werden. Neben der Wildtypsequenz des humanen FSAP-Gens sind verschiedene Nukleotidpolymorphismen bekannt, die in zwei Fällen auch zu einer Änderung der Aminosäuresequenz führen (EP 1 182 258 A1). Der sogenannte Marburg I Polymorphismus (auch MR I Mutation, Allel oder Variante) führt zu einem Gly/Glu Aminosäureaustausch an Position 534 des Proenzyms einschließlich des Signalpeptids (Gly/Glu 534) und resultiert in einer 50-80 %igen Verringerung der Prourokinase-aktivierenden Aktivität, während die Fähigkeit Faktor VII zu aktivieren unverändert erhalten bleibt. Ein weiterer Polymorphismus, der sogenannte Marburg II Polymorphismus (auch MR II Mutation, Allel oder Variante) führt zu einem Glu/Gln Aminosäureaustausch an Position 370 des Proenzyms einschließlich des Signalpeptides (Glu/Gln 370). Die Marburg II Mutation hat jedoch keinen Einfluß auf die Prourokinase-aktivierende Aktivität der FSAP. Ein MR I Polymorphismus wird bei ungefähr 5 % der westeuropäischen Bevölkerung gefunden. Heterozygote Träger des MR I Polymorphismus scheinen ein gegenüber dem Durchschnitt der Bevölkerung höheres Risiko zu tragen, eine Carotis Stenose zu entwickeln [Willeit et al. (2003) Circulation 107: 667-670]. Damit stellt FSAP bzw. die FSAP MR I Variante einen potenziellen Marker zur Erkennung einer Disposition für artherosklerotische Erkrankungen dar.

Die Identifizierung von Personen, die mindestens eine Kopie der FSAP MR I Variante tragen, ist gemäß dem Stand der Technik mit Hilfe verschiedener methodischer Ansätze möglich (siehe EP 952 215 A2 und EP 1 182 258 A1). Eine der bekannten Methoden beruht auf der Bestimmung des Prourokinase Aktivierungspotenzials der FSAP in einer Probe. Dazu wird ein spezifischer Antikörper, der nicht in der Lage ist, zwischen Wildtyp-FSAP und den bekannten FSAP-Varianten zu unterscheiden, an eine Festphase gekoppelt und mit der Probenflüssigkeit inkubiert. Nach Zugabe von Prourokinase und chromogenem Substrat, wird die Menge an umgesetztem Chromogen als Maß für die Prourokinase-aktivierende Aktivität der FSAP bestimmt. Träger des Marburg I Polymorphismus weisen eine um 50-80 % erniedrigte Prourokinase Aktivität auf. Eine verminderte Prourokinase Aktivität kann jedoch auch durch eine geringe FSAP-Konzentration in der Probe bedingt werden. Daher ist es besonders vorteilhaft, nicht nur die Prourokinase Aktivität, sondern zusätzlich auch die FSAP-Antigenkonzentration einer Probe zu bestimmen. Aus dem Stand der Technik sind monoklonale Antikörper bekannt, die den immunologischen Nachweis von FSAP ermöglichen. In EP 1 182 258 A1 sind zwei monoklonale Antikörper beschrieben, die den Hybridomazelllinien DSM ACC2453 bzw. DSM ACC2454 entstammen, welche nach der Immunisierung von Mäusen mit FSAP-Protein gewonnen wurden. Beide Antikörper binden nicht nur das FSAP-Wildtyp-Protein, sondern ebenso die Marburg I und II Varianten. Weitere bekannte FSAP-Antikörper binden gleichermaßen an wildtypisches FSAP wie auch an die bekannten mutanten Varianten, so dass beispielsweise in einem Sandwich-ELISA der Gesamtgehalt an FSAP-Antigen einer Probe bestimmt wird (siehe auch DE 100 23 923 A1). Erst wenn eine verminderte Prourokinase Aktivität zusammen mit einer FSAP-Antigenkonzentration im Normbereich festgestellt wird, ist dies ein konkreter Hinweis auf das Vorliegen einer FSAP MR I Variante.

Allerdings liefert keine der beschriebenen Methoden einen sicheren Beweis für das Vorliegen einer FSAP MR I Variante. Um jedoch im Falle eines verminderten FSAP-Prourokinase-Aktivierungspotenzials zum Beispiel zielgerichtete prophylaktische oder therapeutische Maßnahmen zu ergreifen, ist es durchaus von Wichtigkeit, die Ursache des Funktionsverlustes genau zu diagnostizieren.

Der unzweifelhafte Nachweis des Gly/Glu Aminosäureaustausches an Position 534 des Proenzyms (Gly/Glu 534) ist bislang nur durch die Sequenzierung des korrespondierenden codierenden Bereiches in der genomischen DNA oder der mRNA möglich. Ein G/A Basenaustausch in der genomischen Sequenz, der an Nukleotidposition 1601 der cDNA nachgewiesen werden kann, bildet die genetische Ursache für den FSAP MR I Polymorphismus (siehe EP 1 182 258 A1). Auch wenn die DNA-Sequenz-Analyse zuverlässige Ergebnisse liefert, so besteht seitens des Routinelabors ein Bedarf an etablierten Testverfahren, die möglichst preisgünstig, schnell und sicher sind und die zudem auf vorhandenen Diagnostik-Geräten automatisch abgearbeitet werden können. Vorwiegend bevorzugt sind immunologische Nachweisverfahren bzw. Testassays, da sie die genannten Kriterien erfüllen und in der Labordiagnostik bereits eine breite Anwendung gefunden haben.

Der vorliegenden Erfindung lag also die Aufgabe zugrunde, ein Verfahren bzw. Komponenten bereitzustellen, die den spezifischen Nachweis der FSAP MR I Variante mit Hilfe von Antikörpern ermöglichen.

Die Lösung dieser Aufgabe besteht in der Bereitstellung der in den Ansprüchen beschriebenen erfindungsgemäßen Verfahren und Gegenstände.

Insbesondere wird die Aufgabe durch die Bereitstellung von Antikörpern gelöst, die spezifisch an die FSAP MR I Variante binden, aber nicht an das FSAP-Wildtyp-Protein oder andere mutante Varianten, die nicht durch einen Gly/Glu Aminosäureaustausch an Position 534 des FSAP-Proenzyms gekennzeichnet sind. Diese Antikörper bilden die Basis für den direkten immunologischen Nachweis und die Quantifizierung der FSAP MR I Variante in Plasmaproben heterozygoter Träger oder homozygoter Individuen.

Es hat sich überraschenderweise gezeigt, dass Peptide, die mindestens die Aminosäuresequenz Glu-Cys-Glu-Lys-Arg (SEQ ID NO. 1) enthalten, welche den Aminosäuren 532 bis 536 der FSAP MR I Variante entspricht und somit den Gly/Glu Aminosäureaustausch an Position 534 des Proenzyms (Gly/Glu 534) enthält, als Immunisierungsantigene zur Herstellung FSAP MR I-spezifischer Antikörper besonders geeignet sind. Die erfindungsgemäßen Peptide zeichnen sich dadurch aus, dass der FSAP MR I-spezifische Glu-Rest an Position 534 sowohl N- wie auch C-terminal von mindestens zwei weiteren Aminosäureresten flankiert wird.

Ausdrücklich von der Erfindung ausgeschlossen ist jedoch ein Peptid der Aminosäuresequenz Ser-Thr-Lys-Lys-Leu-Ser-Glu-Cys-Glu-Lys-Arg-Ile-Gly-Asp-Glu-Leu-Asp-Ser-Asn-Met, das in einer Publikation von Shangary & Johnson offenbart ist [Shangary, S. & Johnson, D.E. (2002) Peptides derived from BH3 domaines of Bcl-2 family members: a comparative analysis of inhibition of Bcl-2, Bcl-x_{L} and Bax oligomerization, induction of cytochrome c release, and activation of cell death. Biochemistry 41, 9485-9495; Seite 9487, Tabelle 1]. Shangary & Johnson beschreiben außer dem genannten Peptid noch weitere synthetische Peptide aus der BH3-Domäne von Onkoproteinen der Bcl-2-Familie, die sich aufgrund ihrer Fähigkeit zur Inhibierung von Proten-Protein-Interaktionen zur Regulation apoptotischer Prozesse in Tumorzellen eignen, die aber in keinem technischen Zusammenhang mit der vorliegenden Erfindung stehen.

Im folgenden werden spezifische Ausführungsformen der Erfindung näher erläutert:

Ein Gegenstand dieser Erfindung sind Peptide bestehend aus 5 bis 25 Aminosäuren, bevorzugt aus 5 bis 20 Aminosäuren, ganz besonders bevorzugt aus 10 bis 15 Aminosäuren, die dadurch gekennzeichnet sind, dass sie die Aminosäuresequenz Glu-Cys-Glu-Lys-Arg (SEQ ID NO. 1) enthalten, aber nicht aus der Aminosäuresequenz Ser-Thr-Lys-Lys-Leu-Ser-Glu-Cys-Glu-Lys-Arg-Ile-Gly-Asp-Glu-Leu-Asp-Ser-Asn-Met bestehen. Bevorzugte erfindungsgemäße Peptide sind solche mit der Aminosäuresequenz Tyr-Val-Tyr-Gly-Ile-Val-Ser-Trp-Gly-Leu-Glu-Cys-Glu-Lys-Arg-Pro-Gly-Val-Tyr-Thr-Gln-Val-Thr-Lys-Phe (SEQ ID NO. 2) oder ein Fragment derselben, das mindestens die Aminosäuresequenz Glu-Cys-Glu-Lys-Arg (SEQ ID NO. 1) enthält, insbesondere ein Peptid mit der Aminosäuresequenz Ser-Trp-Gly-Leu-Glu-Cys-Glu-Lys-Arg-Pro-Gly-Val-Tyr (SEQ ID NO. 3).

Der Begriff "Peptide" im Sinne dieser Erfindung umfasst Säureamide, die bei Hydrolyse in Aminosäuren zerfallen, beispielsweise Aminosäurepolymere wie zum Beispiel Polypeptide, Oligopeptide, Proteine oder Proteinfragmente.

Die erfindungsgemäßen Peptide können als Immunisierungsantigen zur Herstellung der erfindungsgemäßen Antikörper oder auch zur affinitätschromatographischen Reinigung der erfindungsgemäßen Antikörper verwendet werden. Ferner können die erfindungsgemäßen Peptide auch in einem in vitro-Verfahren zum quantitativen oder qualitativen Nachweis eines Analyten, bevorzugt der FSAP MR I Variante, verwendet werden. Die erfindungsgemäßen Peptide können auch mit einer Festphase und/oder einer Komponente eines signalbildenden Systems assoziiert sein.

Ein weiterer bevorzugter Gegenstand der Erfindung sind Antikörper, die dadurch gekennzeichnet sind, dass sie an das kennzeichnende Epitop der FSAP MR I Variante binden, d. h. an die Aminosäuresequenz Ser-Trp-Gly-Leu-Glu-Cys-Glu-Lys-Arg-Pro-Gly-Val-Tyr (SEQ ID NO. 3), welche den Aminosäuren 528 - 540 der FSAP MR I Variante entspricht.

Unter dem Begriff "Antikörper" ist im Sinne dieser Erfindung ein Immunglobulin, zum Beispiel ein Immunglobulin der Klasse bzw. Subklasse 1gA, IgD, IgE, IgG₁, 1gG₂ₐ, IgG_{2b}, IgG₃, IgG₄, IgM, zu verstehen. Ein Antikörper weist mindestens eine Bindungsstelle (häufig Paratop genannt) für ein Epitop (häufig auch antigene Determinante genannt) auf einem Antigen oder Hapten auf. Ein solches Epitop ist zum Beispiel durch seine räumliche Struktur und/oder durch das Vorhandensein von polaren und/oder apolaren Gruppen gekennzeichnet. Die Bindungsstelle des Antikörpers ist komplementär zum Epitop. Die Antigen-Antikörper-Reaktion bzw. die Hapten-Antikörper-Reaktion funktioniert nach dem sogenannten "Schlüssel-Schlüsselloch-Prinzip" und ist in der Regel in einem hohen Grad spezifisch, d. h. die Antikörper vermögen kleine Abweichungen in der Primärstruktur, in der Ladung, in der räumlichen Konfiguration und der sterischen Anordnung des Antigens oder Haptens zu unterscheiden. Insbesondere die sogenannten "complementarity determining regions" des Antikörpers tragen zur Bindung des Antikörpers an das Antigen oder Hapten bei.

Der Begriff "Antigene" umfasst monovalente und polyvalente Antigene. Ein polyvalentes Antigen ist ein Molekül oder ein Molekülkomplex, an das/den mehr als ein Immunglobulin gleichzeitig binden kann, während bei einem monovalenten Antigen jeweils nur ein einziger Antikörper zur selben Zeit binden kann. Als Hapten wird üblicherweise ein Molekül bezeichnet, das nicht für sich allein immunogen ist, sondern das zu Immunisierungszwecken üblicherweise an einen Träger gebunden wird.

Unter dem Begriff Antikörper sind im Sinne dieser Erfindung nicht nur komplette Antikörper zu verstehen, sondern ausdrücklich auch Antikörperfragmente, wie z. B. Fab, Fv, F(ab')₂, Fab'; sowie auch chimäre, humanisierte, bi- oder oligospezifische, oder "single chain" Antikörper; des weiteren auch Aggregate, Polymere und Konjugate von Immunglobulinen und/oder deren Fragmente, sofern die Bindungseigenschaften an das Antigen oder Hapten erhalten sind. Antikörperfragmente lassen sich beispielsweise durch enzymatische Spaltung von Antikörpern mit Enzymen wie Pepsin oder Papain herstellen. Antikörperaggregate, -polymere und -konjugate können durch vielfältige Methoden generiert werden, z. B. durch Hitzebehandlung, Umsetzung mit Substanzen wie Glutaraldehyd, Reaktion mit Immunglobulin-bindenden Molekülen, Biotinylierung von Antikörpern und anschließende Reaktion mit Streptavidin oder Avidin, etc.

Bei einem Antikörper im Sinne dieser Erfindung kann es sich um einen monoklonalen oder um einen polyklonalen Antikörper handeln. Der Antikörper kann nach den üblichen Verfahren hergestellt worden sein, z. B. durch Immunisierung des Menschen oder eines Tieres, wie z. B. Maus, Ratte, Meerschweichen, Kaninchen, Pferd, Esel, Schaf, Ziege, Huhn [s. a. Messerschmid (1996) BIOforum 11: 500-502], und anschließender Gewinnung des Antiserums; oder durch die Etablierung von Hybridomazellen und der anschließenden Reinigung der sekretierten Antikörper; oder durch Klonierung und Expression der Nukleotidsequenzen bzw. modifizierter Versionen davon, die die Aminosäuresequenzen kodieren, die für die Bindung des natürlichen Antikörpers an das Antigen und/oder Hapten verantwortlich sind.

Erfindungsgemäße Antikörper sind insbesondere solche Antikörper, die an ein Peptid bestehend aus 5 bis 25 Aminosäuren, bevorzugt aus 5 bis 20 Aminosäuren, ganz besonders bevorzugt aus 10 bis 15 Aminosäuren binden, welches die FSAP MR I-spezifische Aminosäuresequenz Glu-Cys-Glu-Lys-Arg (SEQ ID NO. 1) umfasst. Ganz bevorzugte Antikörper im Sinne dieser Erfindung sind Antikörper, die an das Peptid mit der Aminosäuresequenz Ser-Trp-Gly-Leu-Glu-Cys-Glu-Lys-Arg-Pro-Gly-Val-Tyr (SEQ ID NO. 3) oder an ein Fragment dieses Peptides spezifisch binden, welches mindestens die Aminosäuresequenz Glu-Cys-Glu-Lys-Arg (SEQ ID NO. 1) umfasst.

Besonders bevorzugte Antikörper im Sinne dieser Erfindung sind auch die Antikörper, die von den Hybridomazelllinien
a) MAK (Maus) gegen ECE-KLH 2004 - 9/ 014 (1),
b) MAK (Maus) gegen ECE-KLH 2004 - 9/ 026 (2),
c) MAK (Maus) gegen ECE-KLH 2004 - 35/ 05 (1),
d) MAK (Maus) gegen ECE-KLH 2004 - 34/08 (2) oder
e) MAK (Maus) gegen ECE-KLH 2004 - 151/013 (2)
produziert werden. Die Hybridomazelllinien a) bis c) wurden am 11. August 2004 bei der DSMZ - Deutsche Sammlung von Mikroorganismen und Zellkulturen GmbH, Mascheroder Weg 1 b, 38124 Braunschweig, Deutschland, unter den Eingangsnummern a) DSM ACC2675 b) DSM ACC2676 und c) DSM ACC2674 hinterlegt. Die Hybridomazelllinien d) und e) wurden am 19.05.2005 bei vorgenannter Hinterlegungsstelle unter den Eingangsnummern d) DSM ACC2725 und e) DSM ACC2726 hinterlegt.

Ein weiterer Gegenstand dieser Erfindung sind spezifische Bindungspartner, die an ein Epitop binden, das von einem erfindungsgemäßen Antikörper erkannt wird.

Unter einem "spezifischen Bindungspartner" ist ein Mitglied eines spezifischen Bindungspaares zu verstehen. Bei den Mitgliedern eines spezifischen Bindungspaares handelt es sich um zwei Moleküle, die jeweils mindestens eine zu einer Struktur des anderen Moleküls komplementäre Struktur aufweisen, wobei die beiden Moleküle sich über eine Bindung der komplementären Strukturen zu binden vermögen. Der Begriff Molekül umfasst auch Molekülkomplexe wie z. B. Enzyme, die aus Apo- und Coenzym bestehen, Proteine, die aus mehreren Untereinheiten bestehen, Lipoproteine bestehend aus Protein und Lipiden, etc. Spezifische Bindungspartner können natürlich vorkommende aber auch z. B. mittels chemischer Synthese, mikrobiologischer Techniken und/oder gentechnologischer Verfahren hergestellte Substanzen sein. Zur Illustration des Begriffs spezifischer Bindungspartner, aber nicht als Einschränkung zu verstehend, sind z. B. zu nennen: thyroxinbindendes Globulin, steroidbindende Proteine, Antikörper, Antigene, Haptene, Enzyme, Lektine, Nukleinsäuren, Repressoren, Oligo- und Polynukleotide, Protein A, Protein G, Avidin, Streptavidin, Biotin, Komplementkomponente C1q, nukleinsäurebindende Proteine, etc. Spezifische Bindungspaare sind beispielsweise: Antikörper-Antigen, Antikörper-Hapten, Operator-Repressor, Nuclease-Nukleotid, Biotin-Avidin, Lektin-Polysaccharid, Steroid-steroidbindendes Protein, Wirkstoff-Wirkstoffrezeptor, Hormon-Hormonrezeptor, Enzym-Substrat, lgG-Protein A, komplementäre Oligo- oder Polynukleotide, etc.

Durch die Bereitstellung der erfindungsgemäßen Antikörper ist es dem Fachmann nun möglich, z. B. durch Kompetitionsexperimente [s. a. Peters et al. (1985) Monoklonale Antikörper, Springer Verlag, Kapitel 12.2 "Epitop-Analyse], andere spezifische Bindungspartner, Antikörper ausdrücklich miteingeschlossen, zu identifizieren, die an das Epitop eines erfindungsgemäßen Antikörpers binden. So lassen sich mittlerweile mit Hilfe von Phage Display Bibliotheken, über synthetische Peptiddatenbanken oder mittels "Recombinatorial Antibody Libraries" spezifische Bindungspartner selektieren [Larrick & Fry (1991) Human Antibodies and Hybridomas 2: 172-189].

Gegenstand dieser Erfindung ist auch ein erfindungsgemäßer Antikörper, der mit einer Festphase und/oder einer Komponente eines signalbildenden Systems assoziiert ist.

Der Begriff "Festphase" im Sinne dieser Erfindung beinhaltet einen Gegenstand, der aus porösem und/oder nicht porösem, in der Regel wasserunlöslichem Material besteht und die unterschiedlichsten Formen aufweisen kann, wie z. B. die von Gefäßen, Röhrchen, Mikrotitrationsplatten, Kugeln, Mikropartikeln, Stäbchen, Streifen, Filter- oder Chromatographiepapier, etc. In der Regel ist die Oberfläche der Festphase hydrophil oder kann hydrophil gemacht werden. Die Festphase kann aus den unterschiedlichsten Materialien bestehen wie z. B. aus anorganischen und/oder aus organischen Materialien, aus synthetischen, aus natürlich vorkommenden und/oder aus modifizierten natürlich vorkommenden Materialien. Beispiele für Festphasenmaterialien sind Polymere, wie z. B. Zellulose, Nitrozellulose, Zelluloseacetat, Polyvinylchlorid, Polyacrylamid, vernetzte Dextranmoleküle, Agarose, Polystyrol, Polyethylen, Polypropylen, Polymethacrylat oder Nylon; Keramik, Glas, Metalle, insbesondere Edelmetalle wie Gold und Silber; Magnetit; Mischungen oder Kombinationen derselben; etc.

Auch Zellen, Liposomen oder Phospholipidvesikel sind vom Begriff Festphase miterfasst.

Die Festphase kann einen Überzug aus einer oder mehreren Schichten aufweisen z. B. aus Proteinen, Kohlehydraten, lipophilen Substanzen, Biopolymeren, organischen Polymeren oder Mischungen hiervon, um beispielsweise die unspezifische Bindung von Probenbestandteilen an die Festphase zu unterdrücken oder zu verhindern oder um beispielsweise Verbesserungen zu erreichen hinsichtlich der Suspensionsstabilität von partikulären Festphasen, der Lagerstabilität, der formgebenden Stabilität oder der Resistenz gegen UV-Licht, Mikroben oder sonstige zerstörend wirkende Agenzien.

Bei einem "signalbildenden System" kann es sich um eine oder mehrere Komponenten handeln, wobei es sich wenigstens bei einer Komponente um ein nachweisbares Label handelt. Als Label ist jedes Molekül zu verstehen, das selbst ein Signal produziert oder die Produktion eines Signals induzieren kann, wie z. B. eine fluoreszierende Substanz, eine radioaktive Substanz, ein Enzym oder eine chemilumineszierende Substanz. Das Signal kann beispielsweise anhand der Enzymaktivität, der Lumineszenz, der Lichtabsorption, der Lichtstreuung, der ausgestrahlten elektromagnetischen oder radioaktiven Strahlung oder einer chemischen Reaktion nachgewiesen oder gemessen werden.

Ein Label vermag selbst ein nachweisbares Signal zu erzeugen, so dass keine weiteren Komponenten notwendig sind. Viele organische Moleküle absorbieren ultraviolettes und sichtbares Licht, wodurch diese Moleküle in einen angeregten Energiezustand kommen können und die absorbierte Energie in Form von Licht einer anderen Wellenlänge als der des eingestrahlten Lichts abgeben. Wieder andere Label können direkt ein nachweisbares Signal erzeugen wie z. B. radioaktive Isotope oder Farbstoffe.

Wieder andere Label benötigen zur Signalerzeugung weitere Komponenten, d. h. das signalproduzierende System schließt in einem solchen Fall all die für die Signalbildung benötigten Komponenten mit ein wie z. B. Substrate, Coenzyme, Quencher, Beschleuniger, zusätzliche Enzyme, Substanzen, die mit Enzymprodukten reagieren, Katalysatoren, Aktivatoren, Cofaktoren, Inhibitoren, Ionen etc.

Geeignete Label [s. a. EP 0 515 194 A2; US 5,340,716; US 5,545,834; Bailey et al. (1987) J. Pharmaceutical & Biomedical Analysis 5: 649-658] sind beispielsweise Enzyme einschließlich Meerrettichperoxidase, alkalische Phosphatase, Glukose-6-Phosphatdehydrogenase, Alkoholdehydrogenase, Glucoseoxidase, β-Galactosidase, Luciferase, Urease und Acetylcholinesterase; Farbstoffe; fluoreszierende Substanzen einschließlich Fluoresceinisothiocyanat, Rhodamin, Phycoerythrin, Phycocyanin, Ethidiumbromid, 5-Dimethylaminonapthalen-1-sulfonylchlorid und fluoreszierende Chelate von seltenen Erden; chemilumineszierende Substanzen einschließlich Luminol, Isoluminol, Acridiniumverbindungen, Olefin, Enolether, Enamin, Arylvinylether, Dioxen, Arylimidazol, Lucigenin, Luciferin und Aequorin; Sensitizer einschließlich Eosin, 9,10-Dibromoanthracen, Methylen Blau, Porphyrin, Phthalocyanin, Chlorophyll, Rose Bengal; Coenzyme; Enzymsubstrate; radioaktive Isotope einschließlich ¹²⁵I, ¹³¹I, ¹⁴C, ³H, ³²P, ³³P, ³⁵S, ¹⁴C, ⁵¹Cr, ⁵⁹Fe, ⁵⁷Co und ⁷⁵Se; Partikel einschließlich magnetische Partikel oder Partikel, bevorzugt Latexpartikel, die selbst beispielsweise mit Farbstoffen, Sensitizern, fluoreszierenden Substanzen, chemilumineszierenden Substanzen, Isotopen oder anderen nachweisbaren Labeln markiert sein können; Solpartikel einschließlich Gold- oder Silbersolen; Liposomen oder Zellen, die selbst mit nachweisbaren Labeln markiert sein können; etc.

Ein signalbildendes System kann auch Komponenten umfassen, die bei räumlicher Nähe miteinander in eine nachweisbare Wechselwirkung treten können, z. B. in Form von Energiespendern und Energieempfängern wie beispielsweise Photosensitizer und chemolumineszierende Substanzen (EP 0 515 194 A2), Photosensitizer und Fluorophore (WO 95/06877), radioaktives lod¹²⁵ und Fluorophore [Udenfriend et al. (1985) Proc. Natl. Acad. Sci. 82: 8672-8676], Fluorophore und Fluorophore [Mathis (1993) Clin. Chem. 39: 1953-1959] oder Fluorophore und Fluoreszenz-Quencher (US 3,996,345). Unter einer Wechselwirkung zwischen den Komponenten ist die direkte Übertragung von Energie zwischen den Komponenten, z. B. durch Licht- oder Elektronenstrahlung sowie über kurzlebige reaktive chemische Moleküle, miteingeschlossen. Ferner umfasst sind hiervon auch Vorgänge, bei denen die Aktivität einer Komponente durch eine oder mehrere andere inhibiert oder verstärkt wird, beispielsweise die Hemmung oder Steigerung der Enzymaktivität oder die Hemmung, Steigerung oder Veränderung (z. B. Wellenlängenverschiebung, Polarisation) der von der beeinflussten Komponente ausgesendeten elektromagnetischen Strahlung. Die Wechselwirkung zwischen den Komponenten umfasst auch Enzymkaskaden. In diesem Fall sind die Komponenten Enzyme, von denen mindestens eines das Substrat für ein anderes liefert, so dass eine maximale oder minimale Reakionsgeschwindigkeit der gekoppelten Substratumsetzung resultiert. Eine effektive Wechselwirkung zwischen den Komponenten findet in der Regel statt, wenn diese räumlich benachbart vorliegen, also z. B. innerhalb eines Abstandbereiches von wenigen µm, insbesondere innerhalb eines Abstandbereiches von unter 600 nm, bevorzugt unter 400 nm, ganz besonders bevorzugt von unter 200 nm.

Mikropartikel werden häufig als Festphase und/oder als Label genutzt. Unter dem Begriff "Mikropartikel" sind im Sinne dieser Erfindung Teilchen zu verstehen, die einen ungefähren Durchmesser von wenigstens 20 nm und nicht mehr als 20 µm aufweisen, üblicherweise zwischen 40 nm und 10 µm, bevorzugt zwischen 0,1 und 10 µm, besonders bevorzugt zwischen 0,1 und 5 µm, ganz besonders bevorzugt zwischen 0,15 und 2 µm. Die Mikropartikel können regelmäßig oder unregelmäßig geformt sein. Sie können Kugeln, Spheroide, Kugeln mit mehr oder weniger großen Kavitäten oder Poren darstellen. Die Mikropartikel können aus organischem, aus anorganischem Material oder aus einer Mischung oder Kombination von beiden bestehen. Sie können aus einem porösen oder nicht porösen, einem schwellfähigen oder nicht schwellfähigen Material bestehen. Prinzipiell können die Mikropartikel jegliche Dichte haben, bevorzugt sind jedoch Partikel mit einer Dichte, die der Dichte des Wassers nahe kommt wie etwa 0,7 bis etwa 1,5 g/ml. Die bevorzugten Mikropartikel sind in wässrigen Lösungen suspendierbar und möglichst lange suspensionsstabil. Sie mögen durchsichtig, teilweise durchsichtig oder undurchsichtig sein. Die Mikropartikel können aus mehreren Schichten bestehen wie beispielsweise die sogenannten "core-andshell" Partikel mit einem Kern und einer oder mehreren umhüllenden Schichten.

Der Begriff Mikropartikel umfasst beispielsweise Farbstoffkristalle, Metallsolen, Silica-Partikel, Glaspartikel, Magnetpartikel, Polymerteilchen, Öltropfen, Lipidpartikel, Dextran und Proteinaggregate. Bevorzugte Mikropartikel sind in wässrigen Lösungen suspendierbare und aus wasserunlöslichem Polymermaterial bestehende Partikel, insbesondere aus substituierten Polyethylenen. Ganz besonders bevorzugt sind Latexpartikel z. B. aus Polystyrol, Acrylsäurepolymeren, Methacrylsäurepolymeren, Acrylnitril-Polymeren, Acrylnitril-Butadien-Styrol, Polyvinylacetat-Acrylat, Polyvinylpyridin, Vinylchlorid-Acrylat. Von besonderem Interesse sind Latexpartikel mit reaktiven Gruppen an ihrer Oberfläche wie beispielsweise Carboxyl-, Amino- oder Aldehydgruppen, die eine kovalente Bindung z. B. von spezifischen Bindungspartnern an die Latexpartikel erlauben. Die Herstellung von Latexpartikeln ist beispielsweise in EP 0 080 614, EP 0 227 054 und EP 0 246 446 beschrieben.

Der Begriff "assoziiert" ist breit zu verstehen und umfasst beispielsweise eine kovalente und eine nicht-kovalente Bindung, eine direkte und eine indirekte Bindung, die Adsorption an eine Oberfläche und den Einschluß in eine Vertiefung oder einen Hohlraum, etc. Bei einer kovalenten Bindung sind die Antikörper oder Bindungspartner über eine chemische Bindung an die Festphase oder an das Label gebunden. Beispiele für eine nicht-kovalente Bindung sind die Oberflächenadsorption, der Einschluss in Hohlräume oder die Bindung von zwei spezifischen Bindungspartnern. Neben einer direkten Bindung an die Festphase oder das Label können die Antikörper oder Bindungspartner auch indirekt über spezifische Wechselwirkung mit anderen spezifischen Bindungspartnern an die Festphase oder das Label gebunden sein (s. a. EP 0 411 945 A2). Dies soll anhand von Beispielen näher illustriert werden: Der biotinylierte Antikörper kann über labelgebundenes Avidin an das Label gebunden werden, oder es kann ein Fluorescein-Antikörperkonjugat über festphasengebundene Anti-Fluorescein-Antikörper an die Festphase gebunden werden, oder der Antikörper kann über lmmunglobulin-bindende Proteine an die Festphase oder das Label gebunden werden.

Ein weiterer Gegenstand dieser Erfindung sind erfindungsgemäße Antikörper oder spezifische Bindungspartner die als ein in vitro Diagnostikum oder als ein Bestandteil eines in vitro Diagnostikums verwendet werden. Bei einem in vitro Diagnostikum wird der nachzuweisende Analyt, z. B. die FSAP Marburg I Variante, in einer Probe außerhalb eines lebenden menschlichen oder tierischen Körpers nachgewiesen oder dessen Konzentration oder Menge bestimmt.

Unter einer "Probe" ist im Sinne der Erfindung das Material zu verstehen, das die nachzuweisende Substanz (Beispiele für den Begriff "Analyt" siehe EP 0 515 194 A2, Seiten 8-15) vermutlich enthält. Der Begriff Probe umfasst beispielsweise biologische Flüssigkeiten oder Gewebe insbesondere von Menschen und Tieren wie Blut, Plasma, Serum, Sputum, Exudat, bronchoalveoläre Lavage, Lymphflüsigkeit, Synovialflüssigkeit, Samenflüssigkeit, Vaginalschleim, Feces, Urin, Liquor, Haare, Haut, Gewebeproben oder -schnitte. Ferner werden umfasst Zellkulturproben, pflanzliche Flüssigkeiten oder Gewebe, forensische Proben, Wasser- und Abwasserproben, Nahrungsmittel, Arzneimittel. Gegebenenfalls müssen die Proben vorbehandelt werden, um den Analyten für das Nachweisverfahren zugänglich zu machen oder um störende Probenbestandteile zu entfernen. Solche Vorbehandlung von Proben mag die Abtrennung und/oder Lyse von Zellen beinhalten, das Präzipitieren, die Hydrolyse oder die Denaturierung von Probenbestandteilen wie z. B. Proteinen, die Zentrifugation von Proben, das Behandeln der Probe mit organischen Lösungsmitteln wie z. B. Alkohole, insbesondere Methanol; das Behandeln der Probe mit Detergenzien. Häufig wird die Probe in ein anderes, meistens wässriges, Medium überführt welches mit dem Nachweisverfahren möglichst nicht interferieren soll.

Die erfindungsgemäßen Antikörper können in einem Verfahren zum quantitativen oder qualitativen Nachweis eines Analyten, bevorzugt der FSAP Marburg I Variante, in einer Probe verwendet werden.

Bei einem quantitativen Nachweis wird die Menge, die Konzentration oder die Aktivität (z. B. Enzymaktivität) des Analyten in der Probe gemessen. Von dem Begriff des "quantitativen Nachweises" sind auch semiquantitative Methoden umfasst, die nur die ungefähre Menge, Konzentration oder Aktivität des Analyten in der Probe erfassen oder nur zu einer relativen Mengen-, Konzentrations- oder Aktivitätsangabe dienen können. Unter einem qualitativen Nachweis ist der Nachweis des Vorhandenseins des Analyten in der Probe überhaupt oder das Anzeigen, dass die Konzentration oder Aktivität des Analyten in der Probe unterhalb oder oberhalb eines bestimmten oder mehrerer bestimmter Schwellenwerte liegt, zu verstehen.

Die Erfindung betrifft somit auch Verfahren zum quantitativen oder qualitativen Nachweis eines Analyten, bevorzugt FSAP Marburg I Variante, in einer Probe und geeignete Reagenzien hierfür.

Zum Nachweis von Analyten werden häufig Bindungsteste eingesetzt, bei denen durch eine spezifische Bindung von nachzuweisendem Analyt an analytspezifische Bindungspartner auf die Anwesenheit, Abwesenheit oder Menge des Analyten in einer Probe geschlossen werden kann. Immunoassays oder auch Verfahren, bei denen Oligo- oder Polynukleotide hybridisiert werden, sind Beispiele für Bindungsteste.

Die sogenannten "heterogenen Bindungsteste" sind durch einen oder mehrere Trennungsschritte und/oder Waschschritte gekennzeichnet. Die Trennung kann beispielsweise durch lmmunfällung, Fällung mit Substanzen wie Polyethylenglykol oder Ammoniumsulfat, Filtration, magnetische Abtrennung, Anbindung an eine Festphase erfolgen. Ein solche "Festphase" besteht aus porösem und/oder nicht porösem, in der Regel wasserunlöslichem Material. Sie kann die unterschiedlichsten Formen aufweisen wie z. B. die von Gefäßen, Röhrchen, Mikrotitrationsplatten, Kugeln, Mikropartikeln, Stäbchen, Streifen, Filter- oder Chromatographiepapier, etc. Bei heterogenen Bindungstesten im Sandwichformat ist in der Regel einer der analytspezifischen Bindungspartner an eine Festphase gebunden und dient zur Abtrennung des Bindungskomplexes "Analyt/analytspezifischer Bindungspartner" von der flüssigen Phase, während der andere analytspezifische Bindungspartner zum Nachweis des Bindungskomplexes ein nachweisbares Label, z. B. ein Enzym, ein Fluoreszenz- oder Chemilumineszenzlabel etc. trägt. Man unterteilt diese Testverfahren weiter in sogenannte Einschritt-Sandwich-Teste, bei denen die beiden spezifischen Bindungspartner simultan mit der Probe inkubiert werden und in Zweischritt-Sandwich-Teste, bei denen die Probe zunächst mit dem Festphasenreagenz inkubiert wird und nach einem Trenn- und Waschschritt der festphasengebundene Bindungskomplex aus Analyt und analytspezifischer Bindungspartner mit dem Nachweisreagenz inkubiert wird.

Bei "homogenen Bindungstesten" erfolgt keine Trennung zwischen freien und an den "Analyt/analytspezifischer Bindungspartner"-Komplex gebundenen Komponenten des signalbildenden Systems. Der Testansatz, welcher die analytspezifischen Bindungspartner, die signalbildenden Komponenten und die Probe enthält, wird nach oder sogar während der Bindungsreaktion ohne einen weiteren Trenn- und/oder Waschschritt gemessen und das entsprechende Messsignal bestimmt. Beispiele für homogene Immunoassays [s. a. Boguslaski & Li (1982) Applied Biochemistry and Biotechnology 7: 401-414) sind viele turbidimetrische oder nephelometrische Methoden, wobei die zum Nachweis verwendeten analytspezifischen Bindungspartner mit Latexpartikeln assoziiert sein können, wie z. B. EMIT^{®}-Teste; CEDIA^{®}-Teste; Fluoreszenz-Polarisations-Immunoassays; Luminescent Oxygen Channeling Immunoassays [LOCI^{®}, siehe EP 0 515 194 A2; Ullman et al. (1994) Proc. Natl. Acad. Sci. 91: 5426-5430; Ullman et al. (1996) Clinical Chemistry 42: 1518-1526] etc. Bei einem homogenen Sandwich-Immunoassay, wie z. B. einem nephelometrischen Latextest, werden die Antikörperreagenzien mit der Probe zusammen inkubiert und die Messung des Signals während und/oder nach der Inkubation durchgeführt, ohne dass ein Trenn- oder Waschschritt vor der Messung durchgeführt wird. Mit anderen Worten ausgedrückt: Es folgt keine Trennung des Antikörper-gebundenen Analyten vom freien Analyten oder von Antikörpern, die keinen Analyten gebunden haben.

Die erfindungsgemäßen Antikörper eignen sich besonders zur Verwendung in homogenen Bindungstesten.

Homogene und heterogene Bindungsteste können auch in Form eines sogenannten "Sandwichassays" durchgeführt werden. Hierbei wird der Analyt z. B. bei einem heterogenen Bindungstest von einem Festphasen-assoziierten, analytspezifischen Bindungspartner und einem analytspezifischen Bindungspartner, der mit einer Komponente eines signalbildenden System assoziiert ist, gebunden. Bei Sandwich-Immunoassays können Antikörper oder Antigene oder Haptene die analytspezifischen Bindungspartner bilden.

Eine weitere spezielle Ausführungsform eines heterogenen oder homogenen Bindungstestes ist der "indirekte Immunoassay". Der Analyt ist in diesem Fall ein Antikörper. Einer der analytspezifischen Bindungspartner ist das Antigen oder z. B. die erfindungsgemäßen Peptide oder ein modifiziertes Antigen des nachzuweisenden Antikörpers (= Analyt), und der andere analytspezifische Bindungspartner ist in der Regel ein Immunglobulin-bindendes Protein wie z. B. ein Antikörper, der den nachzuweisenden Antikörper (= Analyt) spezifisch zu binden vermag.

Bei einem homogenen oder heterogenen "kompetitiven Bindungstest" konkurrieren Proben-Analyt und Reagenz-Analyt um die Bindung an eine limitierte Anzahl von analytspezifischen Bindungspartnern. Der Reagenz-Analyt ist beispielsweise ein "modifizierter Analyt", wie z. B. ein gelabelter oder markierter Analyt, ein Analytteilstück wie z. B. die erfindungsgemäßen Peptide oder ein Analytanalogon. Beispiele zur Illustration des Prinzips: (i) Proben-Analyt konkurriert mit Reagenz-Analyt, der mit einer Komponente eines signalbildenden System assoziiert ist, um die Bindung an Festphasen-assoziierte, analytspezifische Bindungspartner oder (ii) Proben-Analyt konkurriert mit Festphasen-assoziiertem Analyt (= Reagenz-Analyt) um die Bindung an analytspezifische Bindungspartner, die mit einer Komponente eines signalbildenden Systems assoziiert sind.

Der Nachweis der FSAP Marburg I Variante mit den erfindungsgemäßen Antikörpern kann auch mit Verfahren erfolgen wie beispielsweise Western Blot, Dot Blot, Immunelektrophorese, Immunfixations-Elektrophorese, Elektroimmundiffusion, Immunpräzipitation, radiale Immundiffusion, Immunfixation, Immunchromatographie, Latex-Agglutination, turbidimetrischer oder nephelometrischer Test, homogener oder heterogener Bindungstest, Ein- oder Zweischritt-Test, Sandwich-Test, indirekter Test, kompetitiver Test, "point-of-care"-Teste, etc. Diese und andere Nachweisverfahren sind beispielsweise in "Labor und Diagnose", ed. L. Thomas, TH-Books Verlagsgesellschaft mbH, Frankfurt, 1998, Kapitel 60 oder in "Laboratory Techniques in Biochemistry and Molecular Biology - An Introduction to Radioimmunoassay and Related Techniques", ed. T. Chard, Elsevier, Amsterdam, 1987, beschrieben.
Der Begriff "point-of-care-Teste" oder "POC-Teste" schließt Teste ein, bei denen kein separates Analyse- oder Messgerät zur Testdurchführung oder Testauswertung benötigt wird. POC-Teste basieren in vielen Fällen auf immunchromatographischen Verfahren, Immunkomplexabtrennungen per Filtration und/oder Immunfixationstechniken. Die POC-Teste sind insbesondere für Messungen vor Ort, z. B. am Krankenbett oder daheim, für den Notarzt und/oder beim niedergelassenen Arzt und weniger für das Großlabor gedacht. POC-Teste können insbesondere auch von Personen, die keine eingehende medizinischtechnische Ausbildung und Erfahrung auf dem Gebiet der Laboratoriumsmedizin haben, durchgeführt werden. Unter dem Begriff "POC-Teste" sind im Sinne dieser Erfindung auch sogenannte Heimteste oder OTC-Teste zu verstehen, die von medizinischen Laien durchgeführt werden dürfen, so wie z. B. die diversen Schwangerschaftsteste, die für den Heimgebrauch vertrieben werden. Andere POC-Teste betreffen beispielsweise den Nachweis von Herzinfarktmarkern, Drogen, Arzneimitteln, Infektions- und Entzündungsmarkern. Bei vielen POC-Testen sind oder werden im Laufe der Testdurchführung spezifische Bindungspartner an oder auf Filter- oder Chromatographiestreifen oder -scheiben assoziiert. Eine positive oder negative Nachweisreaktion kann zum Beispiel mit dem Erscheinen oder Nichterscheinen einer Farbbande in einem bestimmten Testfeld verknüpft sein und/oder dem Erscheinen oder Nichterscheinen eines bestimmten Symbols, z. B. einem "+", einem "-" und/oder der Intensität des jeweiligen Meßsignals.

Ein POC-Test für die FSAP Marburg I Variante kann beispielsweise so aufgebaut sein: Probe und gelabelte Antikörper, die an die FSAP Marburg I Variante, aber nicht an Wildtyp-FSAP oder andere FSAP-Varianten zu binden vermögen, werden auf einen Teststreifen aufgetragen. Geeignete Label sind z. B. gefärbte Latexpartikel, kolloidales Gold, Enzyme etc. Sofern die FSAP Marburg I Variante in der Probe enthalten ist, werden sich FSAP MR I / Antikörper-Komplexe ausbilden. Diese Komplexe bewegen sich z. B. mittels Kapillarkraft in Richtung auf einen Bereich, in dem Antikörper, die an andere FSAP MR I-Epitope zu binden vermögen und die z. B. in Form einer Bande fixiert sind oder im Laufe des Testverfahrens fixiert werden (z. B. über eine Biotin-Avidin-Brücke). Die gelabelten FSAP MR I / Antikörper-Komplexe werden in diesem Bereich gebunden und bilden mit den fixierten Antikörpern einen Sandwichkomplex aus. Die Intensität des Labelsignals ist hier proportional zur FSAP MR I-Probenkonzentration. Bei einem kompetitiven POC-Testverfahren können beispielsweise Antikörper-Fragmente in einem Bereich des Teststreifens fixiert sein oder im Laufe des Testverfahrens fixiert werden. Dieser fixierte Antikörper würde mit der FSAP Marburg I Variante aus der Probe um die Bindung an gelabelte Anti-FSAP MR I -Antikörper kompetitieren. Alternativ können auch fixierte Anti-FSAP Marburg I Variante-Antikörper und gelabeltes FSAP MR I -Protein bzw. die erfindungsgemäßen Peptide für den Aufbau eines kompetitiven FSAP Marburg I Varianten-Testes eingesetzt werden.

Eine besonders bevorzugte Ausführungsform des erfindungsgemäßen Verfahrens ist ein nephelometrischer oder ein turbidimetrischer Test, insbesondere ein solcher Test bei dem erfindungsgemäße Antikörper - bevorzugt an Mikropartikel (insbesondere an Latexpartikel) assoziiert - eingesetzt werden.

Ein anderer erfindungsgemäßer Gegenstand ist ein Testkit, der einen oder mehrere der erfindungsgemäßen Antikörper und/oder Peptide enthält. In einem solchen Kit sind üblicherweise alle oder nur einige Komponenten eines Testes in abgepackter Form enthalten. Die erfindungsgemäßen Antikörper und/oder Peptide können beispielsweise mit einer oder mehreren Festphasen und/oder einer oder mehreren Komponenten eines signalbildenden Systems assoziiert sein. Der Testkit kann beispielsweise Standards, Kontrollen, Kalibratoren sowie andere Reagenzien, wie z. B. Puffer, Waschlösungen, Messsignal-auslösende Lösungen und/oder Enzymsubstrat, Küvetten, Pipetten und/oder Testanweisungen enthalten.

Als Standards, Kontrollen oder Kalibratoren in Verfahren zum quantitativen oder qualitativen Nachweis der FSAP Marburg I Variante mit Hilfe spezifischer Bindungspartner eignen sich rekonstituierbare lyophilisierte Zubereitungen und besonders Flüssigzubereitungen, die entweder eine definierte Menge FSAP Marburg I Protein, nativ oder rekombinant, oder eine definierte Menge eines erfindungsgemäßen Peptides, welches die Aminosäuresequenz Glu-Cys-Glu-Lys-Arg (SEQ ID NO. 1) aufweist, aber nicht aus der Aminosäuresequenz Ser-Thr-Lys-Lys-Leu-Ser-Glu-Cys-Glu-Lys-Arg-lle-Gly-Asp-Glu-Leu-Asp-Ser-Asn-Met besteht, enthalten. Ebenfalls geeignet sind Peptide, die sich aus einem Teil bestehend aus 5 bis 25 Aminosäuren, der die Aminosäuresequenz Glu-Cys-Glu-Lys-Arg (SEQ ID NO. 1) enthält, und einem weiteren Teil bestehend aus 5 bis 15 Aminosäuren, der die Aminosäuresequenz Glu-Glu-Phe-His-Glu (SEQ ID NO. 4) enthält, zusammensetzen. Der letztgenannte Teil derartiger Fusionspeptide besteht bevorzugterweise aus einem Peptid der Aminosäuresequenz Gln-Asp-Leu-Lys-Lys-Glu-Glu-Phe-His-Giu-Gln-Ser-Phe-Arg-Val (SEQ ID NO. 5) oder aus einem Glu-Glu-Phe-His-Glu (SEQ ID NO. 4) - enthaltenden Fragment desselben. Die Aminosäuresequenz Glu-Glu-Phe-His-Glu (SEQ ID NO. 4) entspricht den Aminosäurepositionen 383 bis 387 des FSAP-Proenzyms und repräsentiert ein Epitop, das sowohl im wildtypischen FSAP-Protein wie auch in der FSAP Marburg I Variante vorhanden ist. Da die erfindungsgemäßen Fusionspeptide sowohl von Bindungspartnern mit Spezifität für den FSAP Marburg I Polymorphismus, wie z. B. von den erfindungsgemäßen Antikörpern, als auch von Bindungspartnern mit Spezifität für das Glu-Glu-Phe-His-Glu - enthaltende Epitop des FSAP-Proenzyms, wie z. B. von monoklonalen Antikörpern, die von der Hybridomazelllinie gebildet werden, die unter der Eingangsnummer DSM ACC2453 bei der DSMZ - Deutsche Sammlung von Mikroorganismen und Zellkulturen GmbH in Braunschweig, Deutschland hinterlegt wurde (siehe EP 1 182 258 A1), gebunden werden, eignen sie sich besonders zur Standardisierung, Kalibrierung oder Kontrolle von qualitativen oder quantitativen Sandwich-Testverfahren. Die Fusionspeptide können durch eine entsprechende Peptidsynthese hergestellt werden. Ebenso ist es möglich, das FSAP Marburg I-Epitop und das wildtypische Epitop mit Hilfe von homobifunktionellen Cross-Linkern, wie z. B. Glutardialdehyd oder Bi-NHS-Ester, oder mit Hilfe von heterobifunktionellen Cross-Linkern, wie z. B. N-Hydroxysuccinimid (NHS) -X-Maleimid bzw. NHS-X-Haloacetyl, N-γ-Maleimidobutyryloxysuccinimide Ester (GMBS), N-Succinimidyl(4-iodoacetyl)aminobenzoat (SIAB) und heterobifunktionellen Reagenzien, die eine freie SH-Gruppe generieren, wie z. B. Iminothiolane, N-Succinimidyl S-acetylthioacetat (SATA), N-Succinimidyl 3-(2-pyridyldithio) propionat (SPDP), 4-Succinimidyloxycarbonyl-methyl-α-(2-pyridyldithio) toluene (SMPT) zu verbinden oder die beiden Epitope an einen gemeinsamen Träger, wie z. B. Ovalbumin, Albumin, Keyhole Limpet Hämocyanin (KLH), Bovines Alpha-Lactalbumin , sowie Polymere, wie z. B. Dextran, Polyethylenglykol, Polyacrylamid oder Poly-d-Glutamin-d-Lysin etc. zu konjugieren. Die Peptide können beispielsweise mit Hilfe von Carbodiimid oder Glutaraldehyd an diese Träger gebunden werden oder auch mittels eines heterobifunktionalen Reagenzes, welches auch als Abstandhalter ("Spacer") wirken kann, wie z. B. N-Maleimidobutyryloxysuccinimidester (GBMS). Weitere Beispiele und Kopplungsmethoden siehe auch Wong, S. (1993) Chemistry of Protein Conjugation and Cross-Linking, CRC Press Inc., Boca Raton.

Ein bevorzugter erfindungsgemäßer Testkit enthält an Latexpartikel assoziierte erfindungsgemäße Antikörper und/oder erfindungsgemäße Peptide.

Ein anderer erfindungsgemäßer Testkit enthält ein Plasmaprodukt bestehend aus einem Pool von mindestens fünf, bevorzugterweise von mehr als 20 humanen Plasmen von heterozygoten FSAP Marburg 1-Spendern. Heterozygote Spender können mittels bekannter Screening-Verfahren (siehe EP 1 182 258 A1) und selbstverständlich auch mit Hilfe der erfindungsgemäßen Antikörper identifiziert werden. Ein solcher FSAP Marburg I-Plasmapool kann z. B. als Standard verwendet werden, wobei für den Pool ein Referenzwert definiert wird, wie z. B. 100 % oder 1 PEU/ml (Plasma äquivalente Einheiten). Ein derartiger Pool bzw. verschiedene Verdünnungen des Plasmapools können für die Einstellung von Substandards (z. B. Peptidstandards) oder auch zur Festlegung eines Cut-Off-Wertes und eines eventuell erforderlichen Graubereichs für einen Antigennachweis der FSAP Marburg I-Variante verwendet werden.

Die erfindungsgemäßen Antikörper und Peptide lassen sich auch für die Affinitätschromatographie verwenden. Unter dem Begriff "Affinitätschromatographie" ist eine Methode zur Reinigung und Isolierung von Substanzen, insbesondere Biopolymeren, zu verstehen, die auf der Tatsache beruht, dass viele Substanzen mit für sie spezifischen Bindungspartnern eine selektive, nichtkovalente, reversible Bindung eingehen können. Das Prinzip des Verfahrens besteht darin, dass der spezifische Bindungspartner an eine unlösliche Matrix (z. B. poröse Gläser, Gele auf Agarose-, Cellulose-, Dextran-, Polymer- und Kieselgelbasis) in der Regel kovalent gebunden und in Kontakt mit einer die Substanz enthaltenden Probe gebracht wird. Die gesuchte Substanz wird wegen ihrer spezifischen Wechelswirkung mit dem Matrix-gebundenen spezifischen Bindungspartner immobilisiert und zurückgehalten, während alle anderen in der Probe enthaltenen Substanzen durch Elution abgetrennt werden. Anschließend wird die gesuchte Substanz mit einem geeigneten Elutionsmittel, das die nichtkovalente Bindung zwischen Substanz und spezifischem Bindungspartner aufhebt, von der Matrix abgelöst (s. a. E. Buddecke, 1989, Grundrisse der Biochemie, Walter de Gruyter, Kapitel 7 "Proteine").

Ein anderer Gegenstand dieser Erfindung umfasst erfindungsgemäße Antikörper oder erfindungsgemäße Peptide in einem pharmazeutisch verträglichen, sterilen Injektionsmedium. Unter einem pharmazeutisch verträglichen, sterilen Injektionsmedium ist beispielsweise eine keimfreie, pyrogenfreie Lösung, z. B. Saline oder eine andere Elektrolytlösung, zu verstehen, wie sie üblicherweise zur intravenösen, intramuskulären, intraperitonealen oder subkutanen Verabreichung von Arzneimitteln, Impfstoffen oder Kontrastmitteln verwendet wird.

Wieder ein anderer Gegenstand dieser Erfindung ist die Verwendung der erfindungsgemäßen Antikörper als Diagnostikum oder als Bestandteil eines Diagnostikums.

Ein anderer Gegenstand dieser Erfindung ist ein Verfahren zur Herstellung eines erfindungsgemäßen Antikörpers, welches dadurch gekennzeichnet ist, dass zur Immunisierung ein oder mehrere Peptide bestehend aus 5 bis 25 Aminosäuren, bevorzugt aus 5 bis 20 Aminosäuren, ganz besonders bevorzugt aus 10 bis 15 Aminosäuren, eingesetzt werden, welche die Aminosäuresequenz Glu-Cys-Glu-Lys-Arg (SEQ ID NO. 1) enthalten, aber nicht aus der Aminosäuresequenz Ser-Thr-Lys-Lys-Leu-Ser-Glu-Cys-Glu-Lys-Arg-Ile-Gly-Asp-Glu-Leu-Asp-Ser-Asn-Met bestehen. Besonders bevorzugt werden in diesem erfindungsgemäßen Verfahren als Immunisierungsantigen Peptide mit der Aminosäuresequenz Ser-Trp-Gly-Leu-Glu-Cys-Glu-Lys-Arg-Pro-Gly-Val-Tyr (SEQ ID NO. 3) oder Fragmente die mindestens die Aminosäuresequenz Glu-Cys-Glu-Lys-Arg (SEQ ID NO. 1) umfassen.

Die erfindungsgemäßen Antikörper können auch hergestellt werden durch die Verwendung von natürlich vorkommendem und/oder rekombinantem FSAP MR I-Protein bzw. Fragmenten davon, die den FSAP MR I-spezifischen Polymorphismus enthalten.

Die als Immunisierungsantigen verwendeten Peptide können ungebunden und/oder trägergebunden zur Immunisierung verwendet werden.
Typische Träger sind beispielsweise Proteine, wie z. B. Ovalbumin, Albumin oder Keyhole Limpet Hämocyanin (KLH), oder Polymere, wie z. B. Polyethylenglykol, Polyacrylamid oder Poly-d-Glutamin-d-Lysin. Die Peptide können beispielsweise mit Hilfe von Carbodiimid oder Glutaraldehyd an diese Träger gebunden werden oder auch mittels eines heterobifunktionalen Reagenzes, welches auch als Abstandhalter ("Spacer") wirken kann, wie z. B. N-Maleimidobutyryloxysuccinimidester (GBMS). Weitere Beispiele und Kopplungsmethoden s. a. Wong, S. (1993) Chemistry of Protein Conjugation and Cross-Linking, CRC Press, Inc., Boca Raton.
Eine bevorzugtes Verfahren zur Herstellung der erfindungsgemäßen Peptide, die u. a. als Immunisierungsantigene verwendet werden, ist die Festphasensynthese, wobei eine mehrfache Kopienzahl eines Peptids an einem Lysinkern synthetisiert wird [s. a. Tam J. P. (1988) Proc. Natl. Acad. Sci. USA 85: 5409-5413]. Die Peptidsynthese wir vorzugsweise mit Hilfe von Automaten, wie sie z. B. von Applied Biosystems (USA) angeboten werden, nach einem Standardprotokoll durchgeführt. Derartige mutimere Peptide können weiterhin an ein Trägerprotein gebunden werden.

Das Immunisierungsantigen kann beispielsweise in Phosphat-gepufferter Saline aufgenommen werden und mit Immun Easy Mouse Adjuvans versetzt werden. Diese Emulsion kann dann z. B. intradermal, intraperitoneal und/oder subkutan einem Tier, beispielsweise einem Kaninchen, einer Maus, einer Ratte, einem Meerschweinchen, einem Pferd, einem Esel, einem Schaf, einer Ziege, einem Huhn etc. appliziert werden. Booster-Injektionen, wobei das Immunisierungsantigen auch mit inkomplettem Freund'schen Adjuvans emulgiert sein kann, können helfen, die Immunantwort zu steigern.

Erfindungsgemäße polyklonale Antikörper können aus dem Antiserum der immunisierten Tiere gewonnen werden und können per Affinitätschromatographie über eine Matrix, an die beispielsweise die FSAP Marburg I Variante oder die als Immunisierungsantigen eingesetzten Peptide gebunden wurden, weiter aufgereinigt werden.

Um erfindungsgemäße monoklonale Antikörper zu erzeugen, werden nach den allgemein bekannten Verfahren [s. a. Harlow & Lane (1988) Antibodies: A Laboratory Manual, Cold Spring Harbor Laboratory, Cold Spring Harbor; Peters et al. (1985) Monoklonale Antikörper: Herstellung und Charakterisierung, Springer Verlag] die Immunzellen immunisierter Tiere, wie z. B. einer Maus oder eines Kaninchens, mit Myelomzellen zum Erzeugen von Antikörper produzierenden Hybridomazellen verschmolzen und anschließend geeignete Klone isoliert und vereinzelt. Die Auswahl der die gewünschten monoklonalen Antikörper produzierenden Klone wird mit Hilfe spezifischer Screeningverfahren durchgeführt. Hierbei wird die Bindungsspezifität der in den Zellkulturüberstand abgegebenen Antikörper z. B. an das lmmunisierungsantigen oder an einen etwaigen Träger des Immunisierungsantigens mittels Enzymimmunoassay, Radioimmunoassay und/oder Western Blot überprüft. Hybridome, die erfindungsgemäße Antikörper herstellen, werden klonal vermehrt. Die so gewonnenen Hybridomazelllinien stehen dann für eine dauerhafte Produktion von monoklonalen Antikörpern zur Verfügung. Größere Antikörpermengen lassen sich beispielsweise aus Zellkulturüberstand, insbesondere aus Fermentern oder Rollerkulturen sowie aus Aszites gewinnen.

Je nach gewünschtem Verwendungszweck ist es vorteilhaft nur Teile der Antikörper, wie z. B. Fab-, F(ab')₂-, oder Fab'-Fragmente einzusetzen. Diese können beispielsweise mit den dem Fachmann bekannten enzymatischen Spaltverfahren erzeugt werden [s. a. Harlow & Lane (1988) Antibodies: A Laboratory Manual, Cold Spring Harbor Laboratory, Cold Spring Harbor].

Die Antigenbindungsstellen eines Antikörpers befinden sich in den sogenannten variablen Domänen, die durch die V-Gene kodiert sind. Mit den bekannten gentechnischen Methoden [z. B. Sambrook et al. (1989) Molecular Cloning: A Laboratory Manual, Cold Spring Harbor Laboratory, Cold Spring Harbor, 2nd edition; McCafferty et al. (1990) Nature 348: 552-554] kann auch die entsprechende Nukleinsäuresequenz eines erfindungsgemäßen Antikörpers ermittelt werden sowie dadurch auch die entsprechende Aminosäuresequenz, sofern diese noch nicht per Aminosäuresequenzierung bereits bekannt war. Als Ausgangsmaterial für derartige Analysen können die Hybridomazellen bzw. die Antikörper produzierenden Immunzellen immunisierter Tiere eingesetzt werden.

In Kenntnis der Nuklein- und/oder Aminosäuresequenz können mit Hilfe üblicher gentechnologischer und molekularbiologischer Methoden [s. a. Johnson & Chiswell (1993) Current Opinion in Structural Biology 3: 564-571] dann humanisierte, chimäre, bi- oder oligospezifische Antikörper sowie von der "complementarity determining region" abgeleitete Peptide ("minimal recognition units"), single-chain Fragmente, und/oder funktionelle Fusionsprodukte, z. B. rekombinant hergestellte Antikörper-Enzym-Konstrukte, hergestellt werden [s. a. Larrick & Fry (1991) Human Antibodies and Hybridomas 2: 172-189; Kitano et al. (1986) Appl. Microbiol. Biotechnol. 24: 282-286; Thompson et al. (1986) J. Immunol. Methods 94: 7-12], die an das FSAP MR I-spezifische Epitop, insbesondere an ein erfindungsgemäßes Peptid, binden. Mit solchen unter dem Begriff "Antikörper" eingeschlossenen Peptiden kann beispielsweise eine Verringerung der Immunogenität und/oder eine verstärkte Wirksamkeit bei Verabreichnung als Arzneimittel oder in vivo Diagnostikum erzielt werden, und/oder es ergeben sich Vorteile für den Einsatz als oder in einem in vitro Diagnostikum. Die Antikörper sind auch herstellbar, gegebenenfalls unter Zuhilfenahme gentechnologischer Methoden, in Pilzen wie z. B. Hefezellen [Fischer et al. (1999) Biol. Chem. 380: 825-839; Hiatt et al. (1992) Genetic Engineering 14: 49-64), pflanzlichen, tierischen und prokaryontischen Zellen (s. a. WO 95/25172) sowie isolierten menschlichen Zellen.

Ein weiterer Gegenstand dieser Erfindung sind auch Pilze, tierische, pflanzliche oder prokaryontische Zellen sowie isolierte menschliche Zellen, die einen erfindungsgemäßen Antikörper produzieren. Eine bevorzugte Ausführungsform dieser Erfindung umfasst Hybridomazelllinien, die die erfindungsgemäßen Antikörper produzieren, beispielsweise die Hybridomazelllinien a) ECE-KLH 2004 - 9/ 014, b) ECE-KLH 2004 - 9/ 026, c) ECE-KLH 2004 - 35/ 05, d) ECE-KLH 2004-34/08 und e) ECE-KLH 2004-151/013, die bei der DSMZ - Deutsche Sammlung von Mikroorganismen und Zellkulturen GmbH, Mascheroder Weg 1b, 38124 Braunschweig, Deutschland, mit den Eingangsnummern a) DSM ACC2675, b) DSM ACC2676, c) DSM ACC2674, d) DSM ACC2725 und e) DSM ACC2726 hinterlegt wurden.

Die im folgenden beschriebenen Beispiele dienen der exemplarischen Beleuchtung einzelner Aspekte dieser Erfindung und sind nicht als Einschränkung zu verstehen.

### Beispiele:

### Beispiel 1: Herstellung FSAP Marburg I-spezifischer monoklonaler Antikörper

### 1a) Immunisierung von Mäusen

BALB/c Mäuse wurden jeweils mit 20 µg Immunisierungsantigen [an KLH gebundenes Peptid mit der Aminosäuresequenz Ser-Trp-Gly-Leu-Glu-Cys-Glu-Lys-Arg-Pro-Gly-Val-Tyr (SEQ ID NO. 3)] in Immun Easy Mouse Adjuvans (Qiagen GmbH, Deutschland) intraperitoneal immunisiert. Nach 4 und 8 Wochen erfolgte eine Booster-Injektion mit jeweils 20 µg Immunisierungsantigen ohne Adjuvans. Die letzten 3 Tage vor der Fusion wurden die Mäuse intravenös mit jeweils 10 µg Immunisierungsantigen geboostert.

### 1b) Fusion

Nach dem Töten der Mäuse durch CO₂-Inhalation wurden die Milzen entnommen und Einzelzellsuspensionen in serumfreiem Dulbeccos modifiziertem Eagle Medium (DMEM; PAN Biotech GmbH, Deutschland) hergestellt. Die Zellen wurden zentrifugiert (652 x g) und 2 × in DMEM gewaschen. Anschließend wurde die Zellzahl mittels Trypanblau-Färbung bestimmt. Zu etwa 10⁸ Milzzellen wurden 2 × 10⁷ Myelomzellen (Sp2/0) gegeben. Nach dem Zentrifugieren (360 × g) wurde der Überstand verworfen, 1 ml Polyethylenglycol-Lösung (PEG 4000, Merck Eurolab GmbH, Deutschland; ca. 50 %ig in DMEM) auf das Zellpellet gegeben und nach Resuspension 1 Minute bei 37 °C inkubiert. Anschließend wurden ca. 10 ml DMEM zugegeben und 2 bis 4 Minuten bei Raumtemperatur inkubiert. Die fusionierten Zellen wurden abzentrifugiert (326 × g) und das Pellet in DMEM + 10 % fötalem Kälberserum (Bio Whittaker Europe, Belgien) + HAT-Medium (CC Pro GmbH, Deutschland) resuspendiert und in 24 Well-Zellkulturplatten (Corning Costar GmbH, Deutschland) abgefüllt. Die ungefähre Zellkonzentration betrug 5 × 10⁴ bis 5 × 10⁶ Zellen pro Well.

Nach 2 bis 3 Wochen wurden die entstandenen Zellkolonien (Hybride) entnommen und in neue Kulturplatten überführt.

### 1c) Screening

Die Spezifität der in die Zellkultur abgegebenen Antikörper wurde in einem ersten Testschritt mit Hilfe von Mikrotiterplatten (Nunc GmbH & Co. KG, Deutschland), die mit einem Peptid mit der Aminosäuresequenz Ser-Trp-Gly-Leu-Glu-Cys-Glu-Lys-Arg-Pro-Gly-Val-Tyr (SEQ ID NO. 3) beschichtet waren, getestet.
In jede Vertiefung der Mikrotiterplatte wurden 100 µl Zellkulturüberstand (Verdünnung 1:2) pipettiert und 1 Stunde bei + 15 bis + 25 °C inkubiert. Nach zweimaligem Waschen der Platte mit Waschlösung-POD (OSEW; Dade Behring Marburg GmbH, Deutschland) wurden in jede Vertiefung 100 µl anti-Maus 1gG/F(ab')₂-POD-Konjugat (Dade Behring Marburg GmbH, Deutschland) eingefüllt und 1 Stunde bei + 15 bis + 25 °C inkubiert. Nach weiterem zweimaligen Waschen der Platte wurde in jede Vertiefung 100 µl Chromogen TMB-Lösung (Dade Behring Marburg GmbH, Deutschland) eingefüllt und weitere 30 Minuten bei + 15 bis + 25 °C inkubiert. Nach der Inkubation wurde in jede Vertiefung 100 µl Stopplösung POD (Dade Behring Marburg GmbH, Deutschland) eingefüllt und die Mikrotitterplatte am BEP II (Behring-ELISA-Prozessor II, Dade Behring Marburg GmbH, Deutschland) bei 450 nm ausgewertet.
In einem zweiten Testschritt wurden die Hybride nach Vereinzelung wie oben beschrieben noch einmal im gleichen Testformat überprüft.

### 1d) Klonierung

Einzelne Zellen von Hybriden, die FSAP MR I-spezifische Antikörper produzieren, wurden mit einem Mikromanipulator (Leitz Messtechnik GmbH, Deutschland) kloniert. Kulturüberstände dieser Klone wurden wie unter 1g) beschrieben gereinigt und wie unter 1e), 1h) und 1i) beschrieben näher charakterisiert. Erfindungsgemäße Antikörper, die an das FSAP MR I-spezifische Epitop binden, werden beispielsweise von den Klonen
a) MAK (Maus) gegen ECE-KLH 2004 - 9/ 014 (1),
b) MAK (Maus) gegen ECE-KLH 2004 - 9/ 026 (2),
c) MAK (Maus) gegen ECE-KLH 2004 - 35/ 05 (1),
d) MAK (Maus) gegen ECE-KLH 2004 - 34/08 (2) und
e) MAK (Maus) gegen ECE-KLH 2004 - 151/013 (2)
produziert. Diese Hybridomazelllinien wurden bei der DSMZ Deutsche Sammlung von Mikroorganismen und Zellkulturen GmbH, Mascheroder Weg 1b, 38124 Braunschweig, Deutschland, mit den Eingangsnummern a) DSM ACC2675, b) DSM ACC2676, c) DSM ACC2674, d) DSM ACC2725 und e) DSM ACC2726 hinterlegt.

### 1e) Bestimmung der Antikörpersubklasse

Die Subklasse der Antikörper gegen FSAP Marburg I Variante wurde mittels IsoStrip^{™}-Mouse Monoclonal Antibody Isotyping Kit- der Fa. Boehringer Mannheim, Deutschland bestimmt. Folgende Subklassen wurde ermittelt:
a) MAK (Maus) gegen ECE-KLH 2004 - 9/ 014 (1) - Subklasse : IgG 2a,
b) MAK (Maus) gegen ECE-KLH 2004 - 9/ 026 (2) - Subklasse : IgG 2a,
c) MAK (Maus) gegen ECE-KLH 2004 - 35/ 05 (1) - Subklasse : IgG 2b,
d) MAK (Maus) gegen ECE-KLH 2004 - 34/08 (2) - Subklasse : IgG 1,
e) MAK (Maus) gegen ECE-KLH 2004 - 151/013 (2) - Subklasse : IgG 1.

### 1f) Produktion der Antikörper

Für die Produktion größerer Mengen Antikörper werden die entsprechenden Zellklone in Rollerflaschen (Corning Costar GmbH, Deutschland) überführt, und bis zum gewünschten Endvolumen bei + 37 °C expandiert. Danach wird die Rollerkultur-Suspension zur Entfernung der Zellen über 0,22 µm filtriert. Die jetzt zellfreie Antikörperlösung wird über Ultrafilter (Trenngrenze 30.000 Dalton) ankonzentriert und anschließend aufgereinigt.

### 1g) Reinigung der Antikörper

Die erhaltene Antikörperlösung wird mit 0,14 M Phosphatpuffer pH 8,6 umgepuffert und auf ein mit rProtein A Sepharose^{™} Fast Flow (Amersham Biosciences Europe GmbH, Deutschland) gefüllte Chromatographiesäule aufgetragen (pro 10 mg zu reinigendem Antikörper wird 1 ml rProtein A Sepharose^{™} Fast Flow eingesetzt). Alle nicht gebundenen Komponenten werden durch Waschen der Säule mit 0,14 M Phosphatpuffer pH 8,6 entfernt. Der gebundene Antikörper wird mit 0,1 M Zitronensäure pH 3,0 von der Säule eluiert und gegen 0,05 M Natriumacetat + 0,5 M NaCl + 0,05 M Tris + 0,01 % Natriumazid pH 7,0 dialysiert.

### 1h) Selektion geeigneter Antikörper für einen FSAP Marburg I Sandwich ELISA

Die Reaktion der monoklonalen anti-FSAP Marburg I-Antikörper mit dem FSAP MR I-spezifischen Epitop [Peptid mit der Aminosäuresequenz Ser-Trp-Gly-Leu-Glu-Cys-Glu-Lys-Arg-Pro-Gly-Val-Tyr (SEQ ID NO. 3)] bzw. dem korrespondierenden FSAP Wildtyp-Epitop [Peptid mit der Aminosäuresequenz Ser-Trp-Gly-Leu-Glu-Cys-Gly-Lys-Arg-Pro-Gly-Val-Tyr (SEQ ID NO. 6)] wurde untersucht:
Reaktion mit einem FSAP MR I - spezifischen Peptid:

Als Festphase wird eine Mikrotiterplatte verwendet, die mit einem Peptid der Aminosäuresequenz Ser-Trp-Gly-Leu-Glu-Cys-Glu-Lys-Arg-Pro-Gly-Val-Tyr (SEQ ID NO. 3) beschichtet ist. Die anti-FSAP MR I-Antikörper aus Kulturüberständen werden daran inkubiert. Nach einem Waschschritt wird eine Bindung des Antikörpers an das Peptid durch ein Konjugat, bestehend aus polyklonalen anti-Maus-Antikörpern aus Kaninchen und dem Enzym Peroxidase, mit anschließender Farbreaktion nachgewiesen.

### Reaktion mit einem FSAP-Wildtyp-Peptid:

Als Festphase wird eine Mikrotiterplatte verwendet, die mit einem Peptid der Aminosäuresequenz Ser-Trp-Gly-Leu-Glu-Cys-Gly-Lys-Arg-Pro-Gly-Val-Tyr (SEQ ID NO. 6) beschichtet ist. Die anti-FSAP MR I-Antikörper aus Kulturüberständen werden daran inkubiert. Nach einem Waschschritt wird eine Bindung des Antikörpers an das Peptid durch ein Konjugat, bestehend aus polyklonalen anti-Maus-Antikörpern aus Kaninchen und dem Enzym Peroxidase, mit anschließender Farbreaktion nachgewiesen.

Es wurden solche Antikörper selektiert, die eine Reaktion mit dem Peptid der Aminosäuresequenz Ser-Trp-Gly-Leu-Glu-Cys-Glu-Lys-Arg-Pro-Gly-Val-Tyr (SEQ ID NO. 3) (FSAP Marburg I Variante) zeigten, gleichzeitig aber nicht mit dem Peptid der Aminosäuresequenz Ser-Trp-Gly-Leu-Glu-Cys-Gly-Lys-Arg-Pro-Gly-Val-Tyr (SEQ ID NO. 6) (FSAP Wildtyp) reagierten. Die Eignung dieser Antikörper zur Verwendung als Festphasen Antikörper in einem Sandwich-ELISA mit FSAP MR 1-spezifischem Konjugatantikörper, der mit einem dem Fachmann bekannten Verfahren an Meerrettich-Peroxidase gekoppelt ist (z. B. Nakane-Konjugation), wurde untersucht.

Die Überprüfung der Eignung erfolgte im Sandwich-ELISA, wie im Beispiel 3a) beschrieben. Die wesentlichen Entscheidungskriterien für die Eignung waren eine eindeutige Differenzierung zwischen FSAP Marburg I Variante und FSAP Wildtyp, d. h. hohe Signalgeneration mit Proben, die die FSAP Marburg I Variante tragen und keine oder niedrige Signalgeneration mit FSAP Wildtyp Proben. Bevorzugte Probenart ist Citratplasma. Weitere Kriterien sind die untere Nachweisgrenze, und die Linearität der Kalibrationskurve.

Die von den Klonen
a) MAK (Maus) gegen ECE-KLH 2004 - 9/ 014 (1) (DSM ACC2675),
b) MAK (Maus) gegen ECE-KLH 2004 - 9/ 026 (2) (DSM ACC2676),
c) MAK (Maus) gegen ECE-KLH 2004 - 35/ 05 (1) (DSM ACC2674),
d) MAK (Maus) gegen ECE-KLH 2004 - 34/08 (2) (DSM ACC2725) und
e) MAK (Maus) gegen ECE-KLH 2004 - 151/013 (2) (DSM ACC2726)
produzierten Antikörper zeigten hinsichtlich dieser Kriterien die besten Ergebnisse.

### Beispiel 2: Nachweis der FSAP Marburg I Variante in einer Probe

### 2a) Testverfahren Sandwich-ELISA

Peroxidase-konjugierte anti-FSAP-Antikörper wurden in Kombination mit den erfindungsgemäßen monoklonalen anti-FSAP Marburg I-Antikörper in einem Enzymimmunoassay nach dem Sandwich-Prinzip eingesetzt.
Während der ersten Inkubation bindet sich die in der Probe enthaltene FSAP Marburg I -Antigenvariante - sofern sie vorhanden ist - an die gegen FSAP Marburg I gerichteten, erfindungsgemäßen Antikörper, die an der Oberfläche der Vertiefungen einer Mikrotitrationsplatte fixiert sind. Nach Auswaschen der Vertiefungen werden in einer zweiten Bindungsreaktion Peroxidase-konjugierte anti-FSAP-Antikörper verwendet, die gegen ein beliebiges Epitop der FSAP MR I-Variante bzw. des FSAP-Wildtyp-Proteins gerichtet sind, wie z. B. solche die von einer der Hybridomazelllinien DSM ACC2453 oder DSM ACC2454 gebildet werden (EP 1 182 258 A1). Die überschüssigen Enzym-konjugierten Antikörper werden ausgewaschen. Anschließend wird die gebundene Enzym-Aktivität in den Vertiefungen bestimmt. Die enzymatische Umsetzung von Wasserstoffperoxid und Tetramethylbenzidin wird durch Zusatz von verdünnter Schwefelsäure unterbrochen. Die zur FSAP MR I-Antigenkonzentration proportionale Farbintensität wird photometrisch bei einer Wellenlänge von 450 nm bestimmt und entweder qualitativ über einen Cut Off ausgewertet oder anhand einer Kalibrationskurve aus Standards quantifiziert. Ein solcher erfindungsgemäßer Sandwich-Immunoassay detektiert die FSAP Marburg I Variante spezifisch in nur einem Testverfahren.

Die Ergebnisse sind in Tabelle 1 dargestellt. Die signifikante Farbreaktion in den Proben, die von heterozygoten Probanden mit FSAP Marburg I Polymorphismus stammen, zeigt, dass die Verwendung der erfindungsgemäßen Antikörper eine spezifische und zuverlässige Bestimmung der FSAP Marburg I Variante ermöglicht.

### 2b) Testverfahren LOCI^{®}

Das Messprinzip der LOCI^{®} Technologie beruht auf durch Sauerstoffradikale hervorgerufener Chemilumineszenz. Voraussetzung ist dabei eine durch den Antigen-Antikörper-Komplex geschaffene, räumliche Nähe der reaktiven Komponenten (z. B. zwischen Chemilumineszenz-Latexpartikeln und photosensitiven Latexpartikeln).

Ein Biotin-konjugierter anti-FSAP-Antikörper wurde in Kombination mit den erfindungsgemäßen monoklonalen anti-FSAP Marburg I-Antikörpern in einem homogenen LOCI^{®} nach dem Sandwich-Prinzip eingesetzt. Während der ersten Inkubation bindet die in der Plasmaprobe enthaltene FSAP Marburg I-Antigenvariante - sofern sie vorhanden ist - an die gegen FSAP Marburg I gerichteten, erfindungsgemäßen Antikörper, die an der Oberfläche von Chemilumineszenz-Partikeln fixiert sind, die ein Olefin enthalten. Nach Zugabe des Biotin-konjugierten anti-FSAP-Antikörpers und einer Inkubation von 7 Minuten bei + 37°C werden photosensitive Latexpartikel, auf deren Oberfläche Streptavidin fixiert ist, hinzugegeben. Ist FSAP Marburg I-Antigenvariante in der Probe enthalten, so bilden anti-FSAP MR I-Chemilumineszenz-Partikel, Biotinkonjugierte anti-FSAP-Antikörper und photosensitive Latex-Streptavidin Partikel einen räumlich nahen Komplex. Die photosensitiven Latexpartikel setzen bei Anregung mit Licht einer Wellenlänge von 680 nm kurzlebigen singulären Sauerstoff frei, welcher nun ein Lumineszenz/Fluoreszenz Signal (520-620nm) der durch den Antigen-Antikörper-Komplex gebundenen Chemilumineszenz-Partikel initiiert. Der singuläre Sauerstoff erreicht durch seine Instabilität nur Chemilumineszenz-Partikel, die sich in nächster Nähe (< 200 nm) zu den photosensitiven Latexpartikeln befinden. Das emittierte Licht wird mit einem modifizierten Tecan RSP 150 mit on-board Lumineszenz Detektor Einheit gemessen [Ullman et al. (1994), Proc. Natl. Acad. Sci., 91: 5426-5430; Ullman et al. (1996) Clinical Chemistry, 42: 1518-1526].

### 2c) Nachweis der FSAP Marburg I Variante in Patientenproben

Mit Hilfe des unter 2a) beschriebenen Verfahrens wurden 10 Plasmaproben, mit Hilfe des unter 2b) beschriebenen Verfahrens wurden 84 Plasmaproben von zuvor genotypisierten Probanden untersucht. 5 Proben stammten von Probanden, die in einer Genkopie an der Nukleotidposition 1601 des codierenden Bereichs des FSAP-Gens (wobei 1 das A des Initiationscodons ist) eine G/A Transition aufwiesen und somit heterozygote Träger des FSAP MR I Polymorphismus, der auf Aminosäureebene zu einem Gly/Glu Aminosäureaustausch an Position 534 des Proenzyms (Gly/Glu 534) führt, waren. 5 weitere Proben stammten von Probanden, die an der Nukleotidposition 1601 des codierenden Bereichs des FSAP-Gens keine Mutation, sondern in beiden Genkopien die wildtypische Gensequenz (G) aufwiesen.

Wie aus Tabellen 1 und 2 hervorgeht, ermöglicht die Verwendung eines erfindungsgemäßen Antikörpers in einem Sandwich-ELISA oder LOCI-Assay^{®}, die eindeutige Differenzierung FSAP MR I-positiver und -negativer Proben. Besonders geeignet im LOCI^{®}-Assay sind die monoklonalen Antikörper, die von den Hybridomazelllinien DSM ACC2725 und DSM ACC2726 gebildet werden. Als Cut Off-Wert im LOCI^{®}-Assay wurde ein Signal von 300.000 Counts festgelegt.

**Tabelle 1**

| **Proben Nr.** | **Genotyp an Nukleotidposition 1601** | **OD_{450 nm}** |
|---|---|---|
| 10048 | G/G | 0,011 |
| 10032 | G/G | 0,015 |
| 10033 | G/G | 0,016 |
| 10029 | G/G | 0,021 |
| 10045 | G/G | 0,015 |
| 14942 | G/A | 1,625 |
| 14943 | G/A | 1,545 |
| 7020552 | G/A | 1,723 |
| 10047 | G/A | 1,533 |
| 7020538 | G/A | 1,441 |

**Tabelle 2**

| Proben ID | Genotyp an Nukleotidpos. 1601 | Counts | | Proben ID | Genotyp an Nukleotidpos. 1601 | Counts |
|---|---|---|---|---|---|---|
| **1** | G/G | 8900 | | 43 | G/G | 25400 |
| 2 | **G/A** | **1750000** | | 44 | G/G | 12400 |
| 3 | G/G | 12500 | | 45 | G/G | 31200 |
| 4 | G/G | 4800 | | 46 | G/G | 22200 |
| 5 | G/G | 9800 | | 47 | G/G | 8600 |
| 6 | G/G | 19300 | | **48** | **G/A** | **1821000** |
| 7 | G/G | 22100 | | 49 | G/G | 5300 |
| 8 | G/G | 35100 | | 50 | G/G | 7600 |
| 9 | G/G | 8900 | | 51 | G/G | 9400 |
| 10 | G/G | 7800 | | 52 | G/G | 5900 |
| 11 | G/G | 6600 | | 53 | G/G | 7800 |
| 12 | G/G | 8800 | | 54 | G/G | 8800 |
| 13 | G/G | 4100 | | 55 | G/G | 24500 |
| 14 | G/G | 7700 | | 56 | G/G | 21500 |
| 15 | G/G | 8500 | | 57 | G/G | 27400 |
| 16 | G/G | 4700 | | 58 | G/G | 23300 |
| 17 | G/G | 8900 | | 59 | G/G | 14600 |
| 18 | G/G | 5600 | | 60 | G/G | 7300 |
| 19 | G/G | 7500 | | 61 | G/G | 5600 |
| 20 | G/G | 6900 | | 62 | G/G | 9600 |
| 21 | G/G | 15300 | | 63 | G/G | 6700 |
| 22 | G/G | 14200 | | 64 | G/G | 21400 |
| 23 | G/G | 8600 | | 65 | G/A | **1250000** |
| 24 | G/G | 5600 | | 66 | G/G | 8300 |
| 25 | G/G | 4500 | | 67 | G/G | 7900 |
| 26 | G/G | 7700 | | 68 | G/G | 6900 |
| 27 | G/G | 5900 | | 69 | G/G | 4900 |
| 28 | G/G | 7500 | | 70 | G/G | 8800 |
| 29 | G/G | 7300 | | 71 | G/G | 15400 |
| 30 | G/G | 9600 | | 72 | G/G | 18800 |
| 31 | G/G | 13400 | | 73 | G/G | 7700 |
| 32 | G/G | 7600 | | 74 | G/G | 8300 |
| 33 | G/G | 8500 | | 75 | G/G | 5700 |
| 34 | G/G | 7700 | | **76** | **G/A** | **1479000** |
| 35 | G/G | 6400 | | 77 | G/G | 7800 |
| 36 | G/G | 5600 | | 78 | G/G | 8800 |
| 37 | G/G | 7600 | | 79 | G/G | 9400 |
| 38 | G/G | 9100 | | 80 | G/G | 5800 |
| 39 | G/G | 7300 | | 81 | G/G | 6400 |
| 40 | G/G | 9800 | | 82 | G/G | 7600 |
| 41 | G/G | 4900 | | 83 | G/G | 8200 |
| 42 | G/G | 8500 | | 84 | G/G | 6900 |

## Patentansprüche

1. Peptid bestehend aus 5 bis 25 Aminosäuren, bevorzugt aus 5 bis 20 Aminosäuren, ganz besonders bevorzugt aus 10 bis 15 Aminosäuren, **dadurch gekennzeichnet, dass** es die Aminosäuresequenz Glu-Cys-Glu-Lys-Arg (SEQ ID NO. 1) enthält, aber nicht aus der Aminosäuresequenz Ser-Thr-Lys-Lys-Leu-Ser-Glu-Cys-Glu-Lys-Arg-lie-Gly-Asp-Glu-Leu-Asp-Ser-Asn-Met besteht.

2. Peptid nach Anspruch 1 mit der Aminosäuresequenz Ser-Trp-Gly-Leu-Glu-Cys-Glu-Lys-Arg-Pro-Gly-Val-Tyr (SEQ ID NO. 3) oder ein Fragment desselben, welches mindestens die Aminosäuresequenz Glu-Cys-Glu-Lys-Arg (SEQ ID NO. 1) enthält.

3. Peptid nach Anspruch 1 oder 2 **dadurch gekennzeichnet, dass** es an ein Peptid der Aminosäuresequenz Gln-Asp-Leu-Lys-Lys-Glu-Glu-Phe-His-GluGln-Ser-Phe-Arg-Val (SEQ ID NO. 5) gekoppelt ist oder an ein Fragment desselben, welches mindestens die Aminosäuresequenz Glu-Glu-Phe-His-Glu (SEQ ID NO. 4) enthält.

4. Peptid nach Anspruch 3 **dadurch gekennzeichnet, dass** das Peptid enthaltend die Aminosäuresequenz Glu-Cys-Glu-Lys-Arg (SEQ ID NO. 1) und das Peptid enthaltend die Aminosäuresequenz Glu-Glu-Phe-His-Glu (SEQ ID NO. 4) über heterobifunktionelle oder homobifunktionelle Cross-Linker verbunden sind.

5. Peptid nach Anspruch 1 bis 4 **dadurch gekennzeichnet, dass** dieses mit einer Festphase und/oder einer Komponente eines signalbildenden Systems assoziiert ist.

6. Verwendung eines Peptides nach Anspruch 1 oder 2 zur affinitätschromatographischen Reinigung von Antikörpern.

7. Verwendung eines Peptids nach einem der Ansprüche 1 - 5 in einem in vitro-Verfahren zum quantitativen oder qualitativen Nachweis eines Analyten, bevorzugt FSAP Marburg I Variante.

8. Antikörper **dadurch gekennzeichnet, dass** dieser spezifisch an das kennzeichnende Epitop der FSAP Marburg I Variante und an ein Peptid gemäß einem der Ansprüche 1 und 2 bindet.

9. Antikörper nach Anspruch 8 **dadurch gekennzeichnet, dass** dieser ein monoklonaler oder ein polyklonaler Antikörper ist.

10. Antikörper nach einem der Ansprüche 8-9 **dadurch gekennzeichnet, dass** dieser von einer der Hybridomazelllinien DSM ACC2674, DSM ACC2675, DSM ACC2676, DSM ACC2725 oder DSM ACC2726 produziert wird.

11. Antikörper nach einem der Ansprüche 8-10 **dadurch gekennzeichnet, dass** der Antikörper mit einer Festphase und/oder einer Komponente eines signalbildenden Systems assoziiert ist.

12. Antikörper nach einem der Ansprüche 8 - 10 in einem pharmazeutisch verträglichen, sterilen Injektionsmedium.

13. Verwendung eines Antikörpers nach einem der Ansprüche 8 - 11 in einem Verfahren zum quantitativen oder qualitativen Nachweis eines Analyten, bevorzugt FSAP Marburg I Variante, in einer Probe.

14. Verwendung eines Antikörpers nach einem der Ansprüche 8-11 in einem homogenen Bindungstest zum quantitativen oder qualitativen Nachweis eines Analyten, bevorzugt FSAP Marburg I Variante, in einer Probe.

15. Verwendung eines Antikörpers nach einem der Ansprüche 8 - 11 in der Affinitätschromatographie.

16. Verwendung eines Antikörpers nach einem der Ansprüche 8 - 11 als Diagnostikum oder als Bestandteil eines Diagnostikums.

17. Reagenz enthaltend ein oder mehrere Peptide nach einem der Ansprüche 1 - 5 und/oder einen oder mehrere Antikörper nach einem der Ansprüche 8 - 11.

18. Testkit enthaltend ein Reagenz, das ein oder mehrere Peptide nach einem der Ansprüche 1 - 5 und/oder einen oder mehrere Antikörper nach einem der Ansprüche 8 - 1 enthält.

19. Testkit nach Anspruch 18, das zusätzlich einen FSAP Marburg I Plasmapool enthält.

20. Tierische, pflanzliche, pilzliche oder prokaryontische Zelle sowie isolierte menschliche Zelle **dadurch gekennzeichnet, dass** diese Antikörper nach einem der Ansprüche 8 -10 produziert.

21. Hybridomazelllinie nach Anspruch 20.

22. Hybridomazelllinien nach Anspruch 21, die bei der DSMZ unter den Eingangsnummern DSM ACC2675, DSM ACC2676, DSM ACC2674, DSM ACC2725 und DSM ACC2726 hinterlegt wurden.

23. Verfahren zum quantitativen oder qualitativen Nachweis der Marburg 1 Variante von FSAP in einer Probe unter Verwendung eines oder mehrerer Peptide nach einem der Ansprüche 1 - 5 und/oder eines oder mehrerer Antikörper nach einem der Ansprüche 8 -11.

24. Verfahren nach Anspruch 23 **dadurch gekennzeichnet, dass** Antikörper nach einem der Ansprüche 8 - 11 in Kombination mit Antikörpern verwendet werden, die an Epitope der FSAP Marburg I Variante binden, die zwar spezifisch, aber nicht kennzeichnend für die Marburg Variante I der FSAP sind.

## Claims

1. Peptide comprising from 5 to 25 amino acids, preferably from 5 to 20 amino acids, very particularly preferably from 10 to 15 amino acids, **characterized in that** it contains the amino acid sequence Glu-Cys-Glu-Lys-Arg (SEQ ID NO. 1) but does not consist of the amino acid sequence Ser-Thr-Lys-Lys-Leu-Ser-Glu-Cys-Glu-Lys-Arg-Ile-Gly-Asp-Glu-Leu-Asp-Ser-Asn-Met.

2. Peptide according to Claim 1 having the amino acid sequence Ser-Trp-Gly-Leu-Glu-Cys-Glu-Lys-Arg-Pro-Gly-Val-Tyr (SEQ ID No . 3) or a fragment thereof which contains at least the amino acid sequence Glu-Cys-Glu-Lys-Arg (SEQ ID No. 1).

3. Peptide according to Claim 1 or 2, **characterized in that** it is coupled to a peptide having the amino acid sequence Gln-Asp-Leu-Lys-Lys-Glu-Glu-Phe-His-Glu-Gln-Ser-Phe-Arg-Val (SEQ ID No. 5) or to a fragment thereof which contains at least the amino acid sequence Glu-Glu-Phe-His-Glu (SEQ ID No. 4).

4. Peptide according to Claim 3 **characterized in that** the peptide containing the amino acid sequence Glu-Cys-Glu-Lys-Arg (SEQ ID No. 1) and the peptide containing the amino acid sequence Glu-Glu-Phe-His-Glu (SEQ ID No. 4) are linked to each other by way of heterobifunctional or homobifunctional crosslinkers.

5. Peptide according to Claims 1 to 4, **characterized in that** it is linked to a solid phase and/or a component of a signal-generating system.

6. Use of a peptide according to Claim 1 or 2 for purifying antibodies by means of affinity chromatography.

7. Use of a peptide according to one of Claims 1-5 in an in-vitro method for quantitatively or qualitatively detecting an analyte, preferably FSAP Marburg I variant.

8. Antibody, **characterized in that** it binds specifically to the characterizing epitope of the FSAP Marburg I variant and to a peptide as claimed in one of Claims 1 and 2.

9. Antibody according to Claim 8, **characterized in that** it is a monoclonal antibody or a polyclonal antibody.

10. Antibody according to one of Claims 8-9, **characterized in that** it is produced by one of the hybridoma cell lines DSM ACC2674, DSM ACC2675, DSM ACC2676, DSM ACC2725 and DSM ACC2726.

11. Antibody according to one of Claims 8-10, **characterized in that** it is linked to a solid phase and/or to a component of a signal-generating system.

12. Antibody according to one of Claims 8-10 in a pharmaceutically tolerated, sterile injection medium.

13. Use of an antibody according to one of Claims 8-11 in a method for quantitatively or qualitatively detecting an analyte, preferably FSAP Marburg I variant, in a sample.

14. Use of an antibody according to one of Claims 8-11 in a homogeneous binding test for quantitatively or qualitatively detecting an analyte, preferably FSAP Marburg I variant, in a sample.

15. Use of an antibody according to one of claims 8-11 in affinity chromatography.

16. use of an antibody according to one of Claims 8-11 as diagnostic agent or as a constituent of a diagnostic agent.

17. Reagent which contains one or more peptides according to one of Claims 1-5 and/or one or more antibodies according to one of Claims 8-11.

18. Test kit which comprises a reagent which comprises one or more peptides according to one of Claims 1-5 and/or one or more antibodies according to one of Claims 8-11.

19. Test kit according to Claim 19, which also comprises an FSAP Marburg I plasma pool.

20. Animal, plant, fungal or prokaryotic cell, and also isolated human cell, **characterized in that** it produces antibodies according to one of Claims 8-10.

21. Hybridoma cell line according to Claim 20.

22. Hybridoma cell lines according to Claim 21 which have been deposited in the DSMZ under one of the receipt numbers DSM ACC2675, DSM ACC2676, DSM ACC2674, DSM ACC2725 and DSM ACC2726.

23. Method for quantitatively or qualitatively detecting the Marburg I variant of FSAP in a sample using one or more peptides according to one of Claims 1-5 and/or one or more antibodies according to one of Claims 8-11.

24. Method according to Claim 23, **characterized in that** antibodies according to one of Claims 8-11 are used in combination with antibodies which bind to epitopes of the FSAP Marburg I variant which, while being specific, are not characterizing for the Marburg I variant of FSAP.

## Revendications

1. Peptide constitué de 5 à 25 aminoacides, de préférence de 5 à 20 aminoacides, de façon tout particulièrement préférée, de 10 à 15 aminoacides, **caractérisé en ce qu'**il contient la séquence d'aminoacides Glu-Cys-Glu-Lys-Arg (SEQ ID n° 1), mais ne consiste pas en la séquence d'aminoacides Ser-Thr-Lys-Lys-Leu-Ser-Glu-Cys-Glu-Lys-Arg-Ile-Gly-Asp-Glu-Leu-Asp-Ser-Asn-Met.

2. Peptide selon la revendication 1, ayant la séquence d'aminoacides Ser-Trp-Gly-Leu-Glu-Cys-Glu-Lys-Arg-Pro-Gly-Val-Tyr (SEQ ID n° 3) ou un fragment de celle-ci qui contient au moins la séquence d'aminoacides Glu-Cys-Glu-Lys-Arg (SEQ ID n° 1).

3. Peptide selon la revendication 1 ou 2, **caractérisé en ce qu'**il est couplé à un peptide ayant la séquence d'aminoacides Gln-Asp-Leu-Lys-Lys-Glu-Glu-Phe-His-Glu-Gln-Ser-Phe-Arg-Val (SEQ ID n° 5) ou à un fragment de celle-ci qui contient au moins la séquence d'aminoacides Glu-Glu-Phe-His-Glu (SEQ ID n° 4).

4. Peptide selon la revendication 3, **caractérisé en ce que** le peptide contenant la séquence d'aminoacides Glu-Cys-Glu-Lys-Arg (SEQ ID n° 1) et le peptide contenant la séquence d'aminoacides Glu-Glu-Phe-His-Glu (SEQ ID n° 4) sont reliés par des lieurs *(cross-linkers)* homobifonctionnels ou hétéro-bifonctionnels.

5. Peptide selon la revendication 1 à 4, **caractérisé en ce qu'**il est associé à une phase solide et/ou à un composant d'un système générateur de signal.

6. Utilisation d'un peptide selon la revendication 1 ou 2, pour la purification d'anticorps par chromatographie d'affinité.

7. Utilisation d'un peptide selon l'une quelconque des revendications 1 à 5, dans un procédé in vitro pour la détection qualitative ou quantitative d'un analyte, de préférence la variante Marburg 1 de la FSAP.

8. Anticorps, **caractérisé en ce qu'**il se lie spécifiquement à l'épitope caractérisant de la variante Marburg I de la FSAP et à un peptide selon l'une des revendications 1 et 2.

9. Anticorps selon la revendication 8, **caractérisé en ce qu'**il est un anticorps monoclonal ou un anticorps polyclonal.

10. Anticorps selon l'une des revendications 8-9, **caractérisé en ce qu'**il est produit par l'une des lignées d'hybridomes DSM ACC2674, DSM ACC2675, DSM ACC2676, DSM ACC2725 ou DSM ACC2726.

11. Anticorps selon l'une quelconque des revendications 8 à 10, **caractérisé en ce que** l'anticorps est associé à une phase solide et/ou à un composant d'un système générateur de signal.

12. Anticorps selon l'une quelconque des revendications 8 à 10, dans un milieu injectable stérile, pharmaceutiquement acceptable.

13. Utilisation d'un anticorps selon l'une quelconque des revendications 8 à 11, dans un procédé pour la détection qualitative ou quantitative d'un analyte, de préférence la variante Marburg 1 de la FSAP, dans un échantillon.

14. Utilisation d'un anticorps selon l'une quelconque des revendications 8 à 11, dans un test de liaison homogène pour la détection qualitative ou quantitative d'un analyte, de préférence la variante Marburg 1 de la FSAP, dans un échantillon.

15. Utilisation d'un anticorps selon l'une quelconque des revendications 8 à 11, dans la chromatographie d'affinité.

16. Utilisation d'un anticorps selon l'une quelconque des revendications 8 à 11, en tant qu'agent de diagnostic ou en tant que composant d'un agent de diagnostic.

17. Réactif contenant un ou plusieurs peptides selon l'une quelconque des revendications 1 à 5 et/ou un ou plusieurs anticorps selon l'une quelconque des revendications 8 à 11.

18. Nécessaire d'essai, contenant un réactif qui contient un ou plusieurs peptides selon l'une quelconque des revendications 1 à 5 et/ou un ou plusieurs anticorps selon l'une quelconque des revendications 8 à 11.

19. Nécessaire d'essai selon la revendication 18, qui contient en outre un pool de plasma FSAP Marburg 1.

20. Cellule animale, végétale, fongique ou procaryote, ainsi que cellule humaine isolée, **caractérisée en ce qu'**elle produit des anticorps selon l'une quelconque des revendications 8 à 10.

21. Lignée d'hybridomes selon la revendication 20.

22. Lignées d'hybridomes selon la revendication 21, qui ont été déposées à la DSMZ (Deutsche Sammlung fUr Mikroorganismen und Zellkulturen) sous les numéros d'accès DSM ACC2675, DSM ACC2676, DSM ACC2674, DSM ACC2725 et DSM ACC2726.

23. Procédé pour la détection qualitative ou quantitative de la variante Marburg 1 de la FSAP dans un échantillon, avec utilisation d'un ou de plusieurs peptides selon l'une quelconque des revendications 1 à 5 et/ou d'un ou de plusieurs anticorps selon l'une quelconque des revendications 8 à 11.

24. Procédé selon la revendication 23, **caractérisé en ce qu'**on utilise des anticorps selon l'une quelconque des revendications 8 à 11 en association avec des anticorps qui se lient à des épitopes de la variante Marburg 1 de la FSAP qui sont certes spécifiques, mais non caractérisants de la variante Marburg 1 de la FSAP.
